# EUROPEAN PATENT APPLICATION

(11) **EP 2 502 997 A1**
(43) Date of publication of application: **26.09.2012**
(21) Application number: 11002480.9
(22) Date of filing: 24.03.2011
(51) Int. Cl.: C12N 15/113, A61K 31/713, C12N 5/10

(54) **MicroRNA inhibiting nucleic acid molecule**

(71) Applicant: Ludwig-Maximilians-Universität München, 80539 München (DE)
(72) Inventor: The designation of the inventor has not yet been filed
(74) Representative: Bohmann, Armin K.

(57) **Abstract**

The present invention is related to a nucleic acid molecule consisting of
(a) an miRNA binding site,
wherein the miRNA binding site is capable of binding a targeted miRNA and
wherein the miRNA binding site comprises about 20 to 25 nucleotides;
(b) a 3' flanking nucleotide sequence, wherein the 3' flanking nucleotide sequence is attached to the 3' end of the miRNA binding site and comprises the nucleotide sequence of SEQ ID NO: 1 and
(c) a 5' flanking nucleotide sequence, wherein the 5' flanking nucleotide sequence is attached to the 5' end of the miRNA binding site and comprises the nucleotide sequence of SEQ ID NO: 2.

## Description

### Field of the invention

The present invention is related to a nucleic acid molecule comprising an miRNA binding site wherein the miRNA binding site is capable of binding a targeted miRNA, a nucleic acid library comprising a plurality of the nucleic acid molecule, a vector comprising the nucleic acid molecule, a vector library comprising a plurality of the vector, a cell comprising the nucleic acid molecule and/or the vector, a method for modulating the activtity of an miRNA, a method for modulating the activity of a gene controlled by an miRNA, a method for degrading an miRNA, and a pharmaceutical composition comprising the nucleic acid molecule and/or the vector.

### Background of the Invention

RNA interference also referred to herein as RNAi, is a process of mRNA degradation within living cells and takes part in controlling which genes are active and how active they are. The RNAi pathway is a mechanism which protects the host against pathogens, such as viruses which use double-stranded RNA, also referred to herein as dsRNA, in their life cycles. Two types of small RNA molecules, namely microRNA, also referred to herein as miRNA, and small interfering RNA, also referred to herein as siRNA, are central to RNAi. RNAs are the direct products of genes, and these small RNAs can bind to other specific RNAs, e.g. messenger RNA, also referred to herein as mRNA, and either increase or decrease their activity, e.g. by preventing an mRNA from producing a protein. On the one hand RNAi thus plays a crucial role in directing development, as well as gene expression, on the other hand RNAi was found to play an important role defending cells against pathogenic genes, such as genes derived from viruses.

RNAi could be identified in several eukaryotes, comprising animals, and is initiated by the enzyme Dicer that is cleaving dsRNA molecules into short fragments of about 20 nucleotides in length. The siRNAs derived from long dsRNA precursors differ from miRNAs in that miRNAs, especially those in animals, typically have incomplete base pairing to a target and inhibit the translation of many different mRNAs with similar sequences. In contrast, siRNAs typically base-pair perfectly and induce mRNA cleavage only in a single, specific target (Pillai RS et al., "Repression of protein synthesis by miRNAs: how many mechanisms?", Trends Cell Biol 17 (3): 118-26 (2007)).

Subsequent to the cleavage mediated by Dicer, the siRNA duplex is unwound into two single stranded RNA molecules, namely the passenger strand and the guide strand. The passenger strand will be degraded, and the guide strand is incorporated into the RNA-induced silencing complex, also referred to herein as RISC. The most well-studied outcome is post-transcriptional gene silencing, which occurs when the guide strand base pairs with a complementary sequence of a messenger RNA molecule and induces cleavage by Argonaute, which is the catalytic component of the RISC complex.

MicroRNAs, also referred to herein as miRNAs, are a class of small endogenously expressed small regulatory non-coding RNAs of about 22 nucleotides in length, thought to negatively regulate target mRNAs (Ambros et al.; Nature. 2004 Sep 16;431(7006):350-5. The functions of animal microRNAs). Said negative regulation is mediated by binding of an miRNA binding site to imperfect complementary in the 3'-untranslated regions, also referred to herein as 3'-UTRs, or in the coding sequence, also referred to herein as CDS, of the transcripts, i.e. for example mRNA (Bartel DP et al, Cell 116(2), 281 (2004), "MicroRNAs: genomics, biogenesis, mechanism, and function,"). Thus, miRNAs play a crucial role in the complex network of gene regulation in eukaryotic cells. Being the specificity determining subunit in the RISC, miRNAs are capable of inhibiting the translation of genes. Based on early studies in invertebrates, miRNAs are expected to have roles in developmental regulation and cell differentiation in mammals, and roles for miRNAs in cardiogenesis (Zhao Y et al., Nature. 2005 Jul 14;436(7048):214-20, Serum response factor regulates a muscle-specific microRNA that targets Hand2 during cardiogenesis.) and lymphocyte development (Chen CZ et al., Science. 2004 Jan 2;303(5654):83-6. Epub 2003 Dec 4, MicroRNAs modulate hematopoietic lineage differentiation) have been demonstrated. Several studies suggest a strong connection between miRNA and human cancer (Calin GA et al., Proc.Natl.Acad.Sci U S A, 2004 Aug 10;101(32):11755-60. Epub 2004 Jul 29, MicroRNA profiling reveals distinct signatures in B cell chronic lymphocytic leukemias). Recent reports implicate roles for mammalian miRNAs in metabolic pathways (Esau C et al., J Biol Chem. 2004 Dec 10;279(50):52361-5. Epub 2004 Oct 25, MicroRNA-143 regulates adipocyte differentiation). Furthermore, miRNAs may contribute to destabilization of the RNA molecules bound by the miRNA. Despite the growing list of roles for mammalian miRNAs, most of the hundreds of miRNAs identified in mammals (Griffiths-Jones S et al, Nucleic Acids Res. 2006 Jan 1;34(Database issue):D140-4, miRBase: microRNA sequences, targets and gene nomenclature; Sahi P et al.Nucleic Acids Res. 2006 Jan 1;34(Database issue):D115-8, Argonaute--a database for gene regulation by mammalian microRNAs.) have no reported function. Nevertheless, many miRNAs and their respective targets have been found to be evolutionary highly conserved. One single miRNA may regulate a variety of different genes, in some examples said variety consisting of more than 100 genes (Lewis BP et al., Cell 120(1), 15 (2005), "Conserved seed pairing, often flanked by adenosines, indicates that thousands of human genes are microRNA targets,"). Accordingly, it is generally assumed today that between 30 and 60% of all human genes are regulated by miRNAs (Friedman et al., Genome Res 19, 92-105 (2009). "Most mammalian mRNAs are conserved targets of microRNAs.").

In recent years, the existence of miRNAs has been described for all eukaryotes, except for some yeast species, e.g. Saccharomyces cerevisiae, as well as for some viruses, particularly herpes viruses (Gottwein E and Cullen BR, Cell Host Microbe 3(6), 375 (2008), "Viral and cellular microRNAs as determinants of viral pathogenesis and immunity"). MiRNAs have also been shown to suppress (Lecellier CH et al., Science. 2005 Apr 22;308(5721):557-60, A cellular microRNA mediates antiviral defense in human cells) and enhance (Jopling CL et al, Science. 2005 Sep 2;309(5740):1577-81. Modulation of hepatitis C virus RNA abundance by a liver-specific MicroRNA) levels of viral RNA in cells. In mammals, miRNAs can play diverse roles in viral infection through their capacity to regulate both host and viral genes. Recent reports have demonstrated that specific miRNAs change in expression level upon infection and can impact viral production and infectivity. It is clear that miRNAs are an integral component of viral-host interactions, and it is likely that both host and virus contain mechanisms to regulate miRNA expression and/or activity. Nevertheless, still little is known about the mechanisms by which miRNAs are regulated in viral infection.

Since miRNAs play an important role in almost every important cellular process, miRNAs also provide interesting targets or target structures for new drugs or agents. Thus, how miRNA may be inhibited or "switched-off" most efficiently is currently the subject of intensive research. Previous approaches to inhibit individual miRNAs were mainly based on chemically modified oligonucleotides, such as 2' O-methylated antisense oligonucleotides, sometimes referred to as "AntagomiRs", or Locked Nucleic Acids (LNA) modified antisense oligonucleotides. AntagomiRs and LNAs are small synthetically produced RNA molecules, which may be further chemically modified and which are directly complementary to the respective miRNA. Using AntagomiRs allows for sequence specific shut-off or inhibition of individual miRNAs (Krutzfeldt J. et al., Nucleic Acids Res 35(9), 2885 (2007) "Specificity, duplex degradation and subcellular localization of antagomirs,"; Krutzfeldt J et al., Nature 438(7068), 685 (2005), "Silencing of microRNAs in vivo with 'antagomirs',"). Nevertheless, there are still problems in the therapeutic application thereof, since antagomiRs have to be delivered into the cell for mediating their function on the respective target miRNA. Similarly, Cheng et al. (Cheng et al., 1290-1297, Nucl. Ac. Res., 2005, Ausg. 33, Nr. 4, "Antisense inhibition of human miRNAs and indications for an involvement of miRNA in cell growth and apoptosis",) used antisense-constructs complementary to the miRNA to be inhibited, and Meister et al. (Meister et al. RNA, New York, N.Y. Vol. 10 (3): 544-50, 2004, "Sequence-specific inhibition of microRNA- and siRNA-induced RNA silencing") reported the use of chemically synthesized oligonucleotides for inhibition of miRNA function in cell extracts and mammalian cell cultures. Nevertheless, a major problem relating to the use of chemically synthesized and/or chemically modified oligonucleotides is the limited stability of said oligonucleotides and accordingly the limited long term effect thereof. Accordingly, said oligonucleotides have to be delivered repeatedly in order to achieve a long-term effect. Nevertheless, a repeated administration of said oligonucleotides faces serious concerns with regard to the toxicity of said nucleotides.

A further possibility for specifically inhibiting miRNA mediated regulation provide so called "microRNA sponges" (Ebert MS et al., Nat. Methods 4(9), 721 (2007) "MicroRNA sponges: competitive inhibitors of small RNAs in mammalian cells,"; Ebert MS and Sharp PA, Curr. Biol. 20(19), R858-R861 (2010). "Emerging roles for natural microRNA sponges,"). Said microRNA sponges are generated by cloning multiple binding sites of a specific miRNA, approximately 4 to 10, into the 3'-UTR of a gene. Accordingly, the miRNA sponges provide microRNA inhibitors that can be expressed in cells. This results in transcripts expressed from strong promoters, containing multiple, tandem binding sites to a microRNA of interest. When vectors encoding these sponges are transiently transfected into cultured cells, sponges derepress microRNA targets at least as strongly as chemically modified antisense oligonucleotides by competition of the miRNA sponges and the naturally occuring targets for the miRNAs naturally available in the target cell. As a result miRNA functions and/or miRNA effects may be specifically revoked or abolished. Nevertheless, it will be acknowledged by a person skilled in the art that the application of the above described concept, namely the application of microRNA sponges, also raises serious concerns of safety, particularly due to the repetitive nature of the microRNA sponge element.

Although only few miRNAs have been assigned a physiological function, antisense targeting may be the only possible approach for therapeutically inhibiting miRNAs, since the active molecule, more precisely the miRNA, is a small RNA, which will be challenging to target specifically without using Watson-Crick base pairing. Therefore, identifying optimal chemistries for miRNA inhibition, as well as alternative strategies for mediating said antisense targeting is crucial for effective fictionalization and therapeutic targeting of miRNAs.

The present inventors have found that infection of multiple mouse cell lines with murine cytomegalovirus, herein also referred to as MCMV, a beta-herpesvirus, leads to a rapid degradation of the cellular miRNAs miR-27a and miR-27b (Buck AH et al., RNA 16(2), 307 (2010), "Post-transcriptional regulation of miR-27 in murine cytomegalovirus infection,"). Down-regulation of miR-27a and miR-27b occurred independently from two other miRNAs and analysis of pri-miRNA levels suggest that said regulation occurs post-transcriptionally. Interestingly, miR-27a and miR-27b have antiviral activity against MCMV but it was unclear whether the virus or the host is responsible for said regulating miR-27a and miR-27b.

Recently, Cazalla et al. (Cazalla et al., Science, 328 (5985), 1563, 2010, "Down-regulation of a host microRNA by a Herpesvirus saimiri non-coding RNA") showed that a non-coding RNA, namely HSUR1 of Herpesvirus saimiri, a primate herpes virus and a classic prototype of the gamma(2)-herpesviruses, is leading to the degradation of cellular miR-27a via one single binding site (Steitz J et al., Cold Spring Harb. Perspect. Biol. (2010), "Noncoding RNPs of Viral Origin). Apart from said miR-27a only a single further miRNA, namely miR-20a was degraded by introducing into HSUR1 a nucleotide sequence partially complementary to miR-20a. In any case, however, the effect of these constructs in terms of efficacy of degradation of the targeted miRNAs was rather limited and was less than 5-fold.

Therefore, the problem underlying the present invention is to provide a means which allows for the specific degradation of miRNAs.

It is also a problem underlying the present invention to provide a means which allows for the specific and efficient degradation of miRNAs.

A further problem underlying the present invention is to provide a method for the specific and efficient degradation of miRNAs.

A still further problem underlying the present invention is to provide a method and means for use in such method, whereby the method allows the realization of a sustained prolonged effect on the targeted miRNA.

These and other problems underlying the instant invention are solved by the subject matter of the independent claims. Preferred embodiments may be taken from the dependent claims.

Furthermore, these and other problems underlying the instant invention are solved by the subject matter of the aspects and embodiments of the invention outlined in the following in more detail.

The problem underlying the present invention is solved in a first aspect which is also the first embodiment of the first aspect, by a nucleic acid molecule consisting of
(a) an miRNA binding site,
   wherein the miRNA binding site is capable of binding a targeted miRNA and
   wherein the miRNA binding site comprises about 20 to 25 nucleotides;
(b) a 3' flanking nucleotide sequence, wherein the 3' flanking nucleotide sequence is attached to the 3' end of the miRNA binding site and comprises the nucleotide sequence of SEQ ID NO: 1 and
(c) a 5' flanking nucleotide sequence, wherein the 5' flanking nucleotide sequence is attached to the 5' end of the miRNA binding site and comprises the nucleotide sequence of SEQ ID NO: 2.

In a second embodiment of the first aspect which is also an embodiment of the first embodiment of the first aspect the 3' flanking nucleotide sequence comprises a nucleotide sequence of about 50 to 700 nucleotides, preferably about 50 to 100 nucleotides or 693 nucleotides.

In a third embodiment of the first aspect which is also an embodiment of the first and the second embodiment of the first aspect, preferably of the first embodiment of the first aspect, the 3' flanking nucleotide sequence consists of the nucleotide sequence of SEQ ID NO: 3.

In a fourth embodiment of the first aspect which is also an embodiment of the first, the second and the third embodiment of the first aspect, the 5' flanking nucleotide sequence comprises a nucleotide sequence of about 50 to 250 nucleotides, preferably about 50 to 120 nucleotides or 104 nucleotides.

In a fifth embodiment of the first aspect which is also an embodiment of the first, the second, the third and the fourth embodiment of the first aspect, preferably of the first and the third embodiment of the first aspect, the 5' flanking nucleotide sequence consists of the nucleotide sequence of SEQ ID NO: 4.

In a sixth embodiment of the first aspect which is also an embodiment of the first, the second, the third, the fourth and the fifth embodiment of the first aspect, the nucleic acid molecule according to any one of he first, the second, the third, the fourth and the fifth embodiment of the first aspect further comprises
(d) a coding nucleotide sequence, wherein the coding nucleotide sequence is attached to the 5' end of the 5' flanking nucleotide sequence and wherein the coding sequence codes for an amino acid sequence.

In a seventh embodiment of the first aspect which is also an embodiment of the sixth embodiment of the first aspect, the amino acid sequence is selected from the group comprising an amino acid sequence coding for m169, an amino acid sequence of a green fluorescence protein, an amino acid sequence of a yellow fluorescence protein, an amino acid of a luciferase, and an amino acid sequence of a marker.

In an eighth embodiment of the first aspect which is also an embodiment of the first, the second, the third, the fourth, the fifth, the sixth and the seventh embodiment of the first aspect, the nucleic acid molecule according to any one of the first, the second, the third, the fourth, the fifth, the sixth and the seventh embodiment of the first aspect further comprises
(e) a non-translated transfer nucleotide sequence, which is contained within the coding sequence wherein the coding sequence is attached to the 5' end of the 5' flanking nucleotide sequence and wherein the non-translated transfer nucleotide sequence enhances the transfer of a nucleic acid molecule from a nucleus of a cell into the cytoplasma of the cell or influences the subcellular localization of a nucleic acid molecule within the cell, preferably the nucleic acid molecule is a nucleic acid molecule of any one of the first, the second, the third, the fourth, the fifth, the sixth and the seventh embodiment of the first aspect.

In a ninth embodiment of the first aspect which is also an embodiment of the eighth embodiment of the first aspect, the non-translated transfer nucleotide sequence is an intron sequence, wherein preferably the intron sequence comprises from about 30 to more than 10000 nucleotides, preferentially 30 to 100 nucleotides.

In a tenth embodiment of the first aspect which is also an embodiment of the eighth and the ninth embodiment of the first aspect the non-translated transfer nucleotide sequence comprises a nucleotide sequence of SEQ ID NO: 5.

In an eleventh embodiment of the first aspect which is also an embodiment of the first, the second, the third, the fourth, the fifth, the sixth, the seventh, the eighth, the ninth and the tenth embodiment of the first aspect, the nucleic acid molecule according to any one of the first, the second, the third, the fourth, the fifth, the sixth, the seventh, the eighth, the ninth and the tenth embodiment of the first aspect further comprises
(f) a non-coding nucleotide sequence, wherein the non-coding nucleotide sequence is attached to the 5' end of the coding sequence or the 5' end of the 5' flanking nucleotide sequence.

In a twelfth embodiment of the first aspect which is also an embodiment of the first, the second, the third, the fourth, the fifth, the sixth, the seventh, the eighth, the ninth, the tenth and the eleventh embodiment of the first aspect, the nucleic acid molecule according to any one of the first, the second, the third, the fourth, the fifth, the sixth, the seventh, the eighth, the ninth, the tenth and the eleventh embodiment of the first aspect, further comprises
(g) at least one promoter, wherein the at least one promoter is attached to the 5' of the non-coding nucleotide sequence, the 5' end of the coding sequence, or the 5' end of the 5' flanking group, and wherein the at least one promoter is operable linked to the 5' of the non-coding nucleotide sequence, the 5' end of the coding nucleotide sequence, or the 5' end of the 5' flanking nucleotide sequence so as to allow the transcription of one or several of the nucleotide sequences attached to the 3' end of the promoter.

In a thirteenth embodiment of the first aspect which is also an embodiment of the twelfth embodiment of the first aspect, the promoter comprises a nucleotide sequence according to SEQ ID NO: 6, or a fragment thereof, wherein preferably the fragment is capable of allowing the transcription of one or several of the nucleotide sequences attached to the 3' end of the promoter.

In a fourteenth embodiment of the first aspect which is also an embodiment of the thirteenth embodiment of the first aspect, the promoter fragment comprises a nucleotide sequence according to SEQ ID NO: 7.

In a fifteenth embodiment of the first aspect which is also an embodiment of the first, the second, the third, the fourth, the fifth, the sixth, the seventh, the eighth, the ninth, the tenth, the eleventh, the twelfth, the thirteenth and the fourteenth embodiment of the first aspect, the miRNA binding site comprises
at positions 1 to 7 a nucleotide sequence A which is complementary to the targeted miRNA,
at positions 8 to 16 a nucleotide sequence B within which one, two, three, four, five, six, seven, eight or nine nucleotides are not complementary to the targeted miRNA; and
at positions 17 to the last position of the miRNA binding site a nucleotide sequence which is complementary to the targeted miRNA.

In a sixteenth embodiment of the first aspect which is also an embodiment of the fifteenth embodiment of the first aspect, the complementarity is a perfect complementarity.

In a seventeenth embodiment of the first aspect which is also an embodiment of the fifteenth embodiment of the first aspect, the complementarity is a perfect complementarity at positions 2 to 7 or 3 to 7 of the miRNA binding site.

In an eighteenth embodiment of the first aspect which is also an embodiment of the fifteenth, the sixteenth and the seventeenth embodiment of the first aspect, the complementarity is a perfect complementarity at positions 17 to 20, 17 to 21, 17 to 22, 17 to 23 and 17 to 24 of the miRNA binding site.

In a nineteenth embodiment of the first aspect which is also an embodiment of the first, the second, the third, the fourth, the fifth, the sixth, the seventh, the eighth, the ninth, the tenth, the eleventh, the twelfth, the thirteenth and the fourteenth embodiment of the first aspect, the miRNA binding site comprises in 5'-3' direction a first stretch of consecutive nucleotides, a second stretch of consecutive nucleotides and a third stretch of consecutive nucleotides,
wherein the targeted micro RNA comprises in 3'-5' direction a stretch I of consecutive nucleotides, a stretch II of consecutive nucleotides and a stretch III of consecutive nucleotides,
wherein the first stretch of consecutive nucleotides of the miRNA biding site is essentially complementary to stretch I of the targeted miRNA,
wherein the second stretch of consecutive nucleotides of the miRNA biding site is not complementary to stretch II of the targeted miRNA,
wherein the third stretch of consecutive nucleotides of the miRNA biding site is essentially complementary to stretch III of the targeted miRNA.

In a twentieth embodiment of the first aspect which is also an embodiment of the nineteenth embodiment of the first aspect, the second stretch of consecutive nucleotides of the miRNA binding site is forming a bulge.

In a twenty-first embodiment of the first aspect which is also an embodiment of the twentieth embodiment of the first aspect, the second stretch comprises 3 to 7 nucleotides, preferably 5 nucleotides.

In a twenty-second embodiment of the first aspect which is also an embodiment of the nineteenth, the twentieth and the twenty-first embodiment of the first aspect, the first stretch of consecutive nucleotides of the miRNA biding site comprises 6 to 10 nucleotides, preferably 8 nucleotides.

In a twenty-third embodiment of the first aspect which is also an embodiment of the nineteenth, the twentieth, the twenty-first and the twenty-second embodiment of the first aspect, nucleotides of the first stretch starting from position 1, 2, 3 or 4 of the first stretch are not complementary to stretch I, wherein the remaining nucleotides of the first stretch are complementary to stretch I.

In a twenty-fourth embodiment of the first aspect which is also an embodiment of the nineteenth, the twentieth, the twenty-first, the twenty-second and twenty-third embodiment of the first aspect, the third stretch of consecutive nucleotides of the miRNA biding site comprises 6 to 10 nucleotides, preferably 8 nucleotides.

In a twenty-fifth embodiment of the first aspect which is also an embodiment of the nineteenth, the twentieth, the twenty-first, the twenty-second, twenty-third and the twenty-fourth embodiment of the first aspect, nucleotides of the third stretch starting from position 1, 2, 3, 4, 5, 6 or 7 of the third stretch are complementary to stretch III, wherein the remaining nucleotides of the third stretch are not complementary to stretch III.

In a twenty-sixth embodiment of the first aspect which is also an embodiment of the first, the second, the third, the fourth, the fifth, the sixth, the seventh, the eighth, the ninth, the tenth, the eleventh, the twelfth, the thirteenth, the fourteenth, the fifteenth, the sixteenth, the seventeenth, the eighteenth, the nineteenth, the twentieth, the twenty-first, the twenty-second, the twenty-third, the twenty-fourth and the twenty-fifth embodiment of the first aspect, the nucleic acid molecule consists of a nucleotide sequence according to SEQ ID NO: 8.

In a twenty-seventh embodiment of the first aspect which is also an embodiment of the first, the second, the third, the fourth, the fifth, the sixth, the seventh, the eighth, the ninth, the tenth, the eleventh, the twelfth, the thirteenth, the fourteenth, the fifteenth, the sixteenth, the seventeenth, the eighteenth, the nineteenth, the twentieth, the twenty-first, the twenty-second, the twenty-third, the twenty-fourth and the twenty-fifth embodiment of the first aspect, the nucleic acid molecule consists of an miRNA binding site as defined in any of the first, the second, the third, the fourth, the fifth, the sixth, the seventh, the eighth, the ninth, the tenth, the eleventh, the twelfth, the thirteenth, the fourteenth, the fifteenth, the sixteenth, the seventeenth, the eighteenth, the nineteenth, the twentieth, the twenty-first, the twenty-second, the twenty-third, the twenty-fourth, the twenty-fifth and the twenty-sixth embodiment of the first aspect, a nucleotide sequence according to SEQ ID NO: 9 attached to the 5' end of the miRNA binding site and a nucleotide sequence according to SEQ ID NO: 10 attached to the 3' end of the miRNA binding site.

In a twenty-eighth embodiment of the first aspect which is also an embodiment of the first, the second, the third, the fourth, the fifth, the sixth, the seventh, the eighth, the ninth, the tenth, the eleventh, the twelfth, the thirteenth, the fourteenth, the fifteenth, the sixteenth, the seventeenth, the eighteenth, the nineteenth, the twentieth, the twenty-first, the twenty-second, the twenty-third, the twenty-fourth, the twenty-fifth, the twenty-sixth and the twenty-seventh embodiment of the first aspect, the targeted miRNA is selected from the group comprising human miRNAs, primate miRNAs, mammalian miRNA, avian miRNAs, vertebrate miRNAs and insect miRNAs.

In a twenty-ninth embodiment of the first aspect which is also an embodiment of the first, the second, the third, the fourth, the fifth, the sixth, the seventh, the eighth, the ninth, the tenth, the eleventh, the twelfth, the thirteenth, the fourteenth, the fifteenth, the sixteenth, the seventeenth, the eighteenth, the nineteenth, the twentieth, the twenty-first, the twenty-second, the twenty-third, the twenty-fourth, the twenty-fifth, the twenty-sixth, the twenty-seventh and the twenty-eighth embodiment of the first aspect, the nucleic acid molecule is for use in a method of inhibiting the function of an miRNA.

In a thirtieth embodiment of the first aspect which is also an embodiment of the twenty-ninth embodiment of the first aspect, the inhibiting of the function is by degradation of the targeted the miRNA.

In a thirty-first embodiment of the first aspect which is also an embodiment of the twenty-ninth embodiment of the first aspect, the inhibiting of the function is by competition of the targeted miRNA.

In a thirty-second embodiment of the first aspect which is also an embodiment of the first, the second, the third, the fourth, the fifth, the sixth, the seventh, the eighth, the ninth, the tenth, the eleventh, the twelfth, the thirteenth, the fourteenth, the fifteenth, the sixteenth, the seventeenth, the eighteenth, the nineteenth, the twentieth, the twenty-first, the twenty-second, the twenty-third, the twenty-fourth, the twenty-fifth, the twenty-sixth, the twenty-seventh and the twenty-eighth embodiment of the first aspect, the nucleic acid molecule is for use in a method of inhibiting the function of an miRNA, preferably for use in a method of degrading an miRNA.

In a thirty-third embodiment of the first aspect which is also an embodiment of the twenty-ninth, the thirtieth, the thirty-first and the thirty-second embodiment of the first aspect, the miRNA the function of which is to be inhibited is the targeted miRNA.

The problem underlying the present invention is solved in a second aspect which is also the first embodiment of the second aspect, by a nucleic acid library comprising a plurality of nucleic acid molecules, wherein each nucleic acid molecule of said nucleic acid library is a nucleic acid molecule according to any one of the first, the second, the third, the fourth, the fifth, the sixth, the seventh, the eighth, the ninth, the tenth, the eleventh, the twelfth, the thirteenth, the fourteenth, the fifteenth, the sixteenth, the seventeenth, the eighteenth, the nineteenth, the twentieth, the twenty-first, the twenty-second, the twenty-third, the twenty-fourth, the twenty-fifth, the twenty-sixth, the twenty-seventh, the twenty-eighth, the twenty-ninth, the thirtieth, the thirty-first, the thirty-second and the thirty-third embodiment of the first aspect, wherein the nucleic acid molecules of the nucleic acid library differ in the sequence of the miRNA binding site.

The problem underlying the present invention is solved in a third aspect which is also the first embodiment of the third aspect, by a vector comprising at least one nucleic acid molecule according to any one of the first, the second, the third, the fourth, the fifth, the sixth, the seventh, the eighth, the ninth, the tenth, the eleventh, the twelfth, the thirteenth, the fourteenth, the fifteenth, the sixteenth, the seventeenth, the eighteenth, the nineteenth, the twentieth, the twenty-first, the twenty-second, the twenty-third, the twenty-fourth, the twenty-fifth, the twenty-sixth, the twenty-seventh, the twenty-eighth, the twenty-ninth, the thirtieth, the thirty-first, the thirty-second and the thirty-third embodiment of the first aspect, wherein the vector is different from a vector comprising a murine cytomegalovirus genome.

In a second embodiment of the third aspect which is also an embodiment of the first embodiment of the third aspect, the vector allows the expression of the nucleic acid molecule according to any of the first, the second, the third, the fourth, the fifth, the sixth, the seventh, the eighth, the ninth, the tenth, the eleventh, the twelfth, the thirteenth, the fourteenth, the fifteenth, the sixteenth, the seventeenth, the eighteenth, the nineteenth, the twentieth, the twenty-first, the twenty-second, the twenty-third, the twenty-fourth, the twenty-fifth, the twenty-sixth, the twenty-seventh, the twenty-eighth, the twenty-ninth, the thirtieth, the thirty-first, the thirty-second and the thirty-third embodiment of the first aspect.

In a third embodiment of the third aspect which is also an embodiment of the first and the second embodiment of the third aspect, the vector is for use in a method inhibiting the function of an miRNA wherein preferably the inhibition of the function is by degradation of the targeted miRNA or by competition of the targeted miRNA.

In a fourth embodiment of the first aspect which is also an embodiment of the first, the second and the third embodiment of the third aspect, the vector comprises a vector backbone and at least one nucleic acid molecule according to any one of the first, the second, the third, the fourth, the fifth, the sixth, the seventh, the eighth, the ninth, the tenth, the eleventh, the twelfth, the thirteenth, the fourteenth, the fifteenth, the sixteenth, the seventeenth, the eighteenth, the nineteenth, the twentieth, the twenty-first, the twenty-second, the twenty-third, the twenty-fourth, the twenty-fifth, the twenty-sixth, the twenty-seventh, the twenty-eighth, the twenty-ninth, the thirtieth, the thirty-first, the thirty-second and the thirty-third embodiment of the first aspect.

In a fifth embodiment of the thirds aspect which is also an embodiment of the fourth embodiment of the third aspect, the vector backbone is selected from the group comprising adenovirus, lentivirus, retrovirus, adeno-associated virus and vaccinia virus.

The problem underlying the present invention is solved in a fourth aspect which is also the first embodiment of the fourth aspect, by a vector library comprising a plurality of vectors, wherein each vector of said vector library is a vector according to any one of the first, the second, the third, the fourth and the fifth embodiment of the third aspect, wherein the vectors of the vector library differ in the sequence of the miRNA binding site.

The problem underlying the present invention is solved in a fifth aspect which is also the first embodiment of the fifth aspect, by a cell comprising a nucleic acid molecule according to any one of the first, the second, the third, the fourth, the fifth, the sixth, the seventh, the eighth, the ninth, the tenth, the eleventh, the twelfth, the thirteenth, the fourteenth, the fifteenth, the sixteenth, the seventeenth, the eighteenth, the nineteenth, the twentieth, the twenty-first, the twenty-second, the twenty-third, the twenty-fourth, the twenty-fifth, the twenty-sixth, the twenty-seventh, the twenty-eighth, the twenty-ninth, the thirtieth, the thirty-first, the thirty-second and the thirty-third embodiment of the first aspect and/or a vector according to any one of the first, the second, the third, the fourth and the fifth embodiment of the third aspect.

In a second embodiment of the fifth aspect which is also an embodiment of the first embodiment of the fifth aspect, the cell is a mammalian cell, preferably the cell is selected from the group comprising fibroblast, endothelial cells, epithelial cells, stroma cells and immune cells

In a third embodiment of the fifth aspect which is also an embodiment of the first and the second embodiment of the fifth aspect, the cell is for use in the production of an agent, wherein the agent is selected from the group comprising proteins, viruses, primary metabolites and secondary metabolites virus.

In a fourth embodiment of the fifth aspect which is also an embodiment of the third embodiment of the fifth aspect, the targeted miRNA is an antiviral miRNA or proviral miRNA.

In a fifth embodiment of the fifth aspect which is also an embodiment of the forth embodiment of the fifth aspect, the targeted miRNA is an antiviral miRNA, wherein the antiviral miRNA is selected from the group comprising hsa-miR-24, hsa-miR-103, hsa-miR-199a-3p, and hsa-miR-214.

In a sixth embodiment of the fifth aspect which is also an embodiment of the fourth embodiment of the fifth aspect, the targeted miRNA is an proviral miRNA, wherein the proviral miRNA is selected from the group comprising hsa-miR-122, hsa-miR-30b, hsa-miR-30d, and hsa-miR-93.

The problem underlying the present invention is solved in a sixth aspect which is also the first embodiment of the sixth aspect, by a method for modulating the activity of an miRNA in a cell comprising the step of introducing into the cell a nucleic acid molecule according to any one of the first, the second, the third, the fourth, the fifth, the sixth, the seventh, the eighth, the ninth, the tenth, the eleventh, the twelfth, the thirteenth, the fourteenth, the fifteenth, the sixteenth, the seventeenth, the eighteenth, the nineteenth, the twentieth, the twenty-first, the twenty-second, the twenty-third, the twenty-fourth, the twenty-fifth, the twenty-sixth, the twenty-seventh, the twenty-eighth, the twenty-ninth, the thirtieth, the thirty-first, the thirty-second and the thirty-third embodiment of the first aspect and/or a vector according to any one of the first, the second, the third, the fourth and the fifth embodiment of the third aspect, wherein, preferably, the targeted miRNA of the nucleic acid molecule is the miRNA the activity of which is to be modulated by said method.

The problem underlying the present invention is solved in a seventh aspect which is also the first embodiment of the seventh aspect, by a method for modulating the activity of a gene controlled by an miRNA in a cell comprising the step of introducing into the cell a nucleic acid molecule according to any one of the first, the second, the third, the fourth, the fifth, the sixth, the seventh, the eighth, the ninth, the tenth, the eleventh, the twelfth, the thirteenth, the fourteenth, the fifteenth, the sixteenth, the seventeenth, the eighteenth, the nineteenth, the twentieth, the twenty-first, the twenty-second, the twenty-third, the twenty-fourth, the twenty-fifth, the twenty-sixth, the twenty-seventh, the twenty-eighth, the twenty-ninth, the thirtieth, the thirty-first, the thirty-second and the thirty-third embodiment of the first aspect and/or a vector according to any one of the first, the second, the third, the fourth and the fifth embodiment of the third aspect, wherein, preferably, the targeted miRNA of the nucleic acid molecule is the miRNA which controls the activity of the gene which is to be modulated by said method.

The problem underlying the present invention is solved in an eighth aspect which is also the first embodiment of the eighth aspect, by a method for degrading an miRNA in a cell comprising the step of introducing into the cell a nucleic acid molecule according to any one of the first, the second, the third, the fourth, the fifth, the sixth, the seventh, the eighth, the ninth, the tenth, the eleventh, the twelfth, the thirteenth, the fourteenth, the fifteenth, the sixteenth, the seventeenth, the eighteenth, the nineteenth, the twentieth, the twenty-first, the twenty-second, the twenty-third, the twenty-fourth, the twenty-fifth, the twenty-sixth, the twenty-seventh, the twenty-eighth, the twenty-ninth, the thirtieth, the thirty-first, the thirty-second and the thirty-third embodiment of the first aspect and/or a vector according to any one of the first, the second, the third, the fourth and the fifth embodiment of the third aspect, wherein, preferably, the targeted miRNA of the nucleic acid molecule is the miRNA which is to be modulated by said method.

The problem underlying the present invention is solved in a ninth aspect which is also the first embodiment of the ninth aspect, by a nucleic acid molecule according to any one of the first, the second, the third, the fourth, the fifth, the sixth, the seventh, the eighth, the ninth, the tenth, the eleventh, the twelfth, the thirteenth, the fourteenth, the fifteenth, the sixteenth, the seventeenth, the eighteenth, the nineteenth, the twentieth, the twenty-first, the twenty-second, the twenty-third, the twenty-fourth, the twenty-fifth, the twenty-sixth, the twenty-seventh, the twenty-eighth, the twenty-ninth, the thirtieth, the thirty-first, the thirty-second and the thirty-third embodiment of the first aspect and/or a vector according to any one of the first, the second, the third, the fourth and the fifth embodiment of the third aspect, for use in a method for the treatment of a disease.

In a second embodiment of the ninth aspect which is also an embodiment of the first embodiment of the ninth aspect, the targeted miRNA is a viral miRNA and, preferably, the disease is a viral disease.

In a third embodiment of the ninth aspect which is also an embodiment of the first and the second embodiment of the ninth aspect, the targeted miRNA is a viral miRNA of human herpesvirus, polyomavirus and adenovirus and preferably the disease is a disease involving or being caused by human herpesvirus, polyomavirus and adenovirus.

In a fourth embodiment of the ninth aspect which is also an embodiment of the first, the second and the third embodiment of the ninth aspect, the targeted miRNA is miR-122 of hepatitis C virus and the disease is hepatitis C.

In a fifth embodiment of the ninth aspect which is also an embodiment of the first, the second, and the third embodiment of the ninth aspect, the targeted miRNA is miR-UL112-1 of human cytomegalovirus and the disease is a disease involving or being caused by human cytomegalovirus.

The problem underlying the present invention is solved in a tenth aspect which is also the first embodiment of the tenth aspect, by a pharmaceutical composition comprising a nucleic acid molecule and/or a vector according to any one of the first, the second, the third, the fourth, the fifth, the sixth, the seventh, the eighth, the ninth, the tenth, the eleventh, the twelfth, the thirteenth, the fourteenth, the fifteenth, the sixteenth, the seventeenth, the eighteenth, the nineteenth, the twentieth, the twenty-first, the twenty-second, the twenty-third, the twenty-fourth, the twenty-fifth, the twenty-sixth, the twenty-seventh, the twenty-eighth, the twenty-ninth, the thirtieth, the thirty-first, the thirty-second and the thirty-third embodiment of the first aspect and /or a vector according to any one of the first, the second, the third, the fourth and the fifth embodiment of the third aspect, and a pharmaceutically acceptable carrier.

The present inventors have surprisingly found that a nucleic acid molecule consisting of
(a) an miRNA binding site, wherein the miRNA binding site is capable of binding a targeted miRNA and wherein the miRNA binding site comprises about 20 to 25 nucleotides;
(b) a 3' flanking nucleotide sequence, wherein the 3' flanking nucleotide sequence is attached to the 3' end of the miRNA binding site and comprises the nucleotide sequence of SEQ ID NO: 1 ; and
(c) a 5' flanking nucleotide sequence, wherein the 5' flanking nucleotide sequence is attached to the 5' end of the miRNA binding site and comprises the nucleotide sequence of SEQ ID NO: 2 allows for the specific and selective interaction with a targeted miRNA. Typically, because of this interaction the targeted miRNA is no longer available so as to exert its function. More specifically, the targeted miRNA is no longer available to act by reducing the synthesis of the product of the gene targeted by said miRNA. More specifically, the present inventors have found that the nucleic acid molecule of the invention is highly active in the degradation of the targeted miRNA and can be retargeted to other targeted miRNA by inserting a nucleotide sequence into the miRNA bidning site which is partially complementary to such other targeted miRNA.

The nucleic acid molecule of the invention may be an RNA or a DNA. In case the nucleic acid molecule of the invention is a DNA, the Us indicated in the nucleotides sequences according to the various SEQ ID NOs are actually Ts.

Without wishing to be bound by any theory the 3' flanking nucleotide sequence as well as the 5' flanking nucleotide sequence seem to act such that the miRNA binding site is presented, in particular under physiological conditions as a single-stranded structure. Such single stranded structure preferably allows the interaction of the miRNA binding site and of the nucleic acid molecule of the present invention, respectively, with the targeted miRNA. It is assumed by the instant inventors that the energy released by the interaction of the miRNA with its target has to provide the energy required to resolve the secondary and tertiary, respectively, structure of the miRNA binding site within the nucleic acid molecule according to the present invention.

It will be understood by a person skilled in the art that both the 3' flanking nucleotide sequence and the 5' flanking nucleotide sequence provide for an ultimately three-dimensional scaffold. Such three-dimensional scaffold arranges the various moieties of the nucleic acid molecule according to the present invention so that the above rearrangement of the same as described above becomes possible by being energetically favorable. In the light thereof it will be acknowledged by a person skilled in the art that the specific sequences of the 3' flanking nucleotide sequence and of the 5' flanking nucleotide sequence can be modified or replaced under the proviso that the thus mutated or replaced sequences still allow for an at least functionally similar or structurally similar two-dimensional or three-dimensional scaffold.

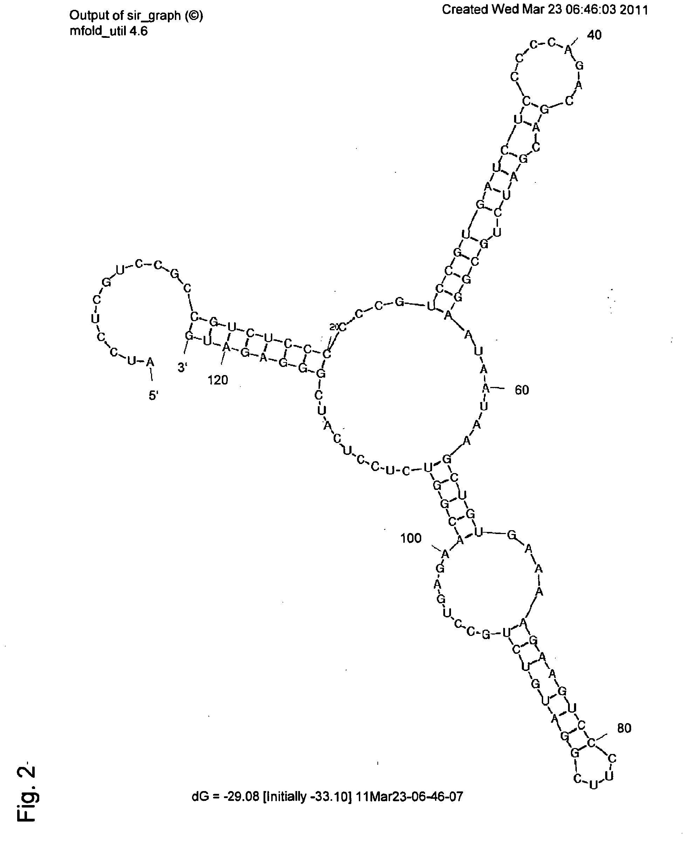

Such functionally similar or structurally similar two dimensional scaffold can be generated by the use of software calculating such scaffold based on energetic considerations. One example for such software is M-FOLD. An example for the scaffold is disclosed and discussed herein in Fig. 2 In accordance therewith a 3' flanking structure used in accordance with the present invention is a 3' flanking structure which forms the same or identical two-dimensional scaffold as predicted by said software based on the nucleotide sequence of the 3' flanking nucleotide sequence as specifically disclosed herein, such as, e.g., SEQ ID NO: 1 or SEQ ID NO: 3. The same applies equally to the 5' flanking nucleotide sequence, whereby the specifically disclosed 5'flanking nucleotide sequence are, e.g. SEQ ID NO: 2 or SEQ ID NO: 4.

It is within the present invention that the 3' flanking nucleotide sequence comprises a nucleotide sequence of SEQ ID: 2 or its 3'-5' inversion, and the 5' nucleotide sequence comprises a nucleotide sequence of SEQ ID NO: 1 or its 3'-5' inversion.

In the embodiment of the nucleic acid molecule of the present invention the 3' flanking nucleotide sequence comprises a nucleotide sequence of about 50 to 700 nucleotides still containing the nucleotide sequence according to SEQ ID NO: 1 or a functional fragment thereof. In a preferred embodiment the length of the 3' flanking nucleotide sequence is about 50 to 100 nucleotides, preferably still containing a nucleotide sequence according to SEQ ID NO: 1 or a functional fragment thereof. It will be understood by a person skilled in the art that other lengths of the 3' flanking nucleotide sequence are possible, whereby, typically, such other lengths still provide for the activity of the nucleic acid molecule of the present invention as described herein, in particular being capable of degrading the targeted miRNA and allowing for retargeting to other targeted miRNA by inserting a nucleotide sequence into the miRNA binding site which is partially complementary to such other targeted miRNA.

In the embodiment of the nucleic acid molecule of the present invention the 5' flanking nucleotide sequence comprises a nucleotide sequence of about 50 to 250 nucleotides still containing the nucleotide sequence according to SEQ ID NO: 2 or a functional fragment thereof. In a preferred embodiment the length of the 5' flanking nucleotide sequence is about 50 to 120 nucleotides, preferably still containing a nucleotide sequence according to SEQ ID NO: 1 or a functional fragment thereof. It will be understood by a person skilled in the art that other lengths of the 5' flanking nucleotide sequence are possible, whereby, typically, such other lengths still provide for the activity of the nucleic acid molecule of the present invention as described herein, in particular being capable of degrading the targeted miRNA and allowing for retargeting to other targeted miRNA by inserting a nucleotide sequence into the miRNA binding site which is partially complementary to such other targeted miRNA.

In a preferred embodiment the term "a functional fragment" of any element or moiety of the nucleic acid molecule of the invention such as the 3' flanking nucleotide sequence, the 5' flanking nucleotide sequence, the coding sequence, the non-translated transfer nucleotide sequence and the non-coding sequence is a fragment which still provides a or the function of the respective full-length nucleotide sequence.

It will be understood by person skilled in the art that the amino acid sequence encoded by the coding nucleotide sequence of the nucleic acid molecule of the invention is, basically, not limited, particularly not limited in terms of the activity or function of such amino acid sequence. A particularly preferred amino acid sequence is one which codes for a marker protein. Preferably a marker protein is one the presence of which in a cell results in a change of the characteristics of the cell which allows the detection or recognition of such cell. Nonlimiting examples thereof comprise fluorescent proteins such as yellow fluorescent protein or green fluorescent protein, or luciferases, which are known in the art.

It is within the present invention that the coding sequence of the nucleic acid molecule of the invention contains a further non-translated sequence which is also referred to herein as a non-translated transfer nucleotide sequence. Preferably said non-translated transfer nucleotide sequence is capable of enhancing the nuclear export, influences subcellular localization and/or enhances translation of a coding nucleotide sequence. In connection therewith nuclear export means the transport of a nucleic acid from the nucleus of a cell, preferably a mammalian cell, into the cytoplasma. The influencing of the subcellular localization of a nucleic acid can be such that the nucleic acid is recruited to ribosomes or the endoplasmatic reticulum. The enhancing of the translation of a coding nucleotide sequence preferably means that either or both the amount of the translation product or the kinetics of translation is increased. It will be understood by a person skilled in the art that these effects of the non-translated transfer nucleotide sequence are exerted in particular onto the nucleic acid molecule of the invention containing such non-translated transfer nucleotide sequence. In a preferred embodiment this non-translated transfer nucleotide sequence is an intron. Introns are known in the art and basically any intron be used in connection with the present invention, particularly if such intron shows at least one of said features, i.e. enhancing the nuclear export, influencing subcellular localization and/or enhancing translation of a coding nucleotide sequence.

Such non-translated transfer nucleotide sequence has typically length of 30 to more than 10000 nucleotides in length. Preferably, the length of the non-translated transfer nucleotide sequence as part of the nucleic acid molecule of the invention is 30 to 100 nucleotides. Functional aspects of introns are, for example, described in Reed R and Cheng H, TREX, SR proteins and export of mRNA, Curr Opin Cell Biol. 2005 Jun;17(3):269-73. (Review).

As to the positioning of the non-translated transfer nucleotide sequence within the coding sequence it will be understood that it may be positioned at any position as long as the function of the nucleic acid molecule of the present invention is still maintained. In a preferred embodiment where the nucleic acid molecule of the invention comprises a coding sequence comprising a nucleotide sequence according to SEQ ID NO: 13, the non-translated transfer nucleotide sequence of SEQ ID NO: 5 is inserted after position 382 of the nucleotide sequence according to SEQ ID NO: 13. In connection therewith it is to be noted that any reference to a position is made on the convention that the first nucleotide is the nucleotide at the 5' end of a nucleotide sequence.

The non-coding nucleotide sequence of the nucleic acid molecule of the invention preferably acts like a 5' UTR as present in a transcript comprising a coding sequence. This means that also in the instant case a non-coding nucleotide sequence of the nucleic acid molecule of the invention is a nucleotide sequence which enhances RNA stability and/or increases efficiency of translation of a coding region attached at the 3' en of the 5' UTR. Insofar the specific nucleotide sequence of the non-coding nucleotide sequence of the nucleic acid molecule of the invention is not necessarily limited to the specific respective sequences disclosed herein

The nucleic acid molecule according to the invention comprises as a further element or moiety at least one promoter. The at least one promoter may be attached to the 5' end of any one of the other elements of the nucleic acid molecule according to the invention. Accordingly and in some embodiments, a promoter is attached to the 5' of the non-coding nucleotide sequence, the 5' end of the coding nucleotide sequence, or the 5' end of the 5' flanking group. Typically, the at least one promoter is operable linked to the 5' of the non-coding nucleotide sequence, the 5' end of the coding nucleotide sequence, or the 5' end of the 5' flanking nucleotide sequence so as to allow the transcription of one or several of the nucleotide sequences attached to the 3' end of the promoter, under the proviso that the respective nucleotide sequence is present in the nucleic acid molecule according to the invention. For example, in case the nucleic acid molecule according to the invention does not contain a non-coding nucleotide sequence but does contain a coding nucleotide sequence, the promoter is attached to the 5' end of the coding nucleotide sequence.

In principle, any promoter can be used which allows the expression of the nucleic acid molecule of the invention in a cell, preferably in the cell into which the nucleic acid molecule of the invention is to be introduced or in which the nucleic acid molecule of the invention is the be effective, more particularly is to inhibit the function of activity of the miRNA targeted by the nucleic acid molecule of the invention. In an embodiment the promoter is a strong promoter which provides a large amount of transcript of the nucleic acid molecule of the invention.

It is also within the present invention that the promoter may actually be a promoter fragment. A promoter fragment is suitable as a promoter for the nucleic acid molecule of the invention if it still exhibits the capacity to act as a promoter and more preferably provides for a transcript of the nucleic acid molecule of the invention under the control of such promoter fragment.

Furthermore, it is within the present invention that the nucleic acid molecule of the invention comprises more than one promoter, whereby such more than one promoter is linked with either the same or a different element or moiety, i.e. a nucleotide sequence as contained in the nucleic acid molecule of the invention.

It will be acknowledged by a person skilled in the art that the term that one nucleotide sequence or a promoter is attached to the 5' end of another nucleotide sequence, preferably means that the last nucleotide, i.e. the nucleotide forming the last nucleotide at or of the 3' end of the one nucleotide sequence or of the promoter is attached to the first nucleotide, i.e. the nucleotide forming the first nucleotide at or of the 5' end of the another nucleotide. The attachment is preferably a covalent bond and more preferably a phosphodiester bond between the respective two nucleotides.

It is within the present invention that apart from the specific promoter described herein by its SEQ ID NO also other promoters, preferably strong promoters, like HCMV IE promoter, EF1 promoter, HSV thymidine kinase promoter may be used in connection with the present invention as a promoter of the nucleic acid molecule.

The miRNA binding site of the nucleic acid molecule of the invention has preferably a length of about 20 to 25 nucleotides. It will be acknowledged by a person skilled in the art that in an embodiment of the invention the miRNA binding site is base pairing with a target miRNA. It is also within the present invention that such base pairing is not a base pairing wherein each and any nucleotide of the miRNA binding site is base pairing with a nucleotide of the targeted miRNA. If there is a base pairing between one or several of the nucleotides of the miRNA binding site and of the targeted miRNA (which may also be referred to herein as the target miRNA), it is also described herein as the one or several nucleotides being complementary to each other. The base pairing may be either a Watson-Crick base pairing or any other form of base pairing such as wobble base pairing and Hoogsteen base pairing, whereby Watson-Crick base pairing is preferred. The base pairing and thus complementarity between the miRNA binding site and the targeted miRNA may be complete or incomplete. In an embodiment the complementarity is complete over one or several stretches of nucleotides of the miRNA binding site and the targeted miRNA and is incomplete over one or several, preferably one stretch of nucleotides of the miRNA binding site and the targeted miRNA. Such incomplete complementarity may result in the formation of a bulge between the miRNA binding site and the targeted miRNA. Such bulge may be as little as on nucleotide and as big as 7 nucleotides. The bulge may be symmetrical such that both the miRNA binding site and the targeted miRNA provide equal numbers of nucleotides involved in the formation of the bulge; alternatively, the bulge may be asymmetrical such that the miRNA binding site and the targeted miRNA provide different numbers of nucleotides involved in the formation of the bulge. A preferred bulge is formed by 5 and 6 nucleotides, whereby 5 nucleotides are preferably provided by the miRNA binding site and 6 nucleotides are provided by the targeted miRNA.

In an embodiment of the invention the most 5'end located nucleotides of the miRNA biding site and the most 3' end located nucleotides of the miRNA binding site are not complementary to the targeted miRNA. In an embodiment of the nucleic acid molecule of the invention the nucleotide forming the 5'end of the miRNA binding site and/or the nucleotide forming the 3' end of the miRNA binding site of the miRNA are not complementary to the targeted miRNA. In an embodiment of the nucleic acid molecule of the invention the two nucleotides forming the 5'end of the miRNA binding site and/or the nucleotide forming the 3' end of the miRNA binding site of the miRNA are not complementary to the targeted miRNA. In an embodiment of the nucleic acid molecule of the invention the nucleotide forming the 5'end of the miRNA binding site and/or the two nucleotides forming the 3' end of the miRNA binding site of the miRNA are not complementary to the targeted miRNA. In an embodiment of the nucleic acid molecule of the invention the two nucleotides forming the 5'end of the miRNA binding site and/or the two nucleotides forming the 3' end of the miRNA binding site of the miRNA are not complementary to the targeted miRNA.

The targeted miRNA may in principle be any miRNA. Preferred miRNAs which can be targeted by the method of the invention and are thus a targeted nucleic acid, are human miRNAs, primate miRNAs, mammalian miRNA, avian miRNAs, vertebrate miRNAs and insect miRNAs. Methods for the identification of miRNAs and antiviral miRNAs are, for example, described in Santhakumar et al. (Proc Natl Acad Sci USA. 2010 Aug 3;107(31):13830-5. Epub 2010 Jul 19. Combined agonist-antagonist genome-wide functional screening identifies broadly active antiviral microRNAs. Santhakumar D, Forster T, Laqtom NN, Fragkoudis R, Dickinson P, Abreu-Goodger C, Manakov SA, Choudhury NR, Griffiths SJ, Vermeulen A, Enright AJ, Dutia B, Kohl A, Ghazal P, Buck AH).

It is within the present invention that in an embodiment any nucleotide sequence disclosed herein, in particular disclosed by its SEQ ID NO, encompasses also those nucleotide sequences which have a homology of at least 80%, 85%, 90%, 95%, 96%, 97%, 98% or 99% relative thereto. Preferable such a homologous nucleotide sequence has the same or a similar function as the nucleotide sequence disclosed herein, in particular disclosed by its SEQ ID NO, preferably in terms of the function being a function as required in connection with the functioning of the nucleic acid molecule of the invention.

An aspect of the invention is a nucleic acid library comprising a plurality of nucleic acid molecules forming the elements of the library, wherein each nucleic acid molecule of said nucleic acid library is a nucleic acid molecule according to the invention, wherein the nucleic acid molecules of the nucleic acid library differ in the sequence of the miRNA binding site. It will be acknowledged by a person skilled in the art that each nucleic acid molecule being an element of the library may be present as a single copy or as several copies.

A further aspect of the invention is a vector comprising at least one nucleic acid molecule of the invention. Such vector is also referred to herein as the vector if the invention. It is also within the present invention that the vector contains multiple copies of the at least one nucleic acid molecule. It is also within the present invention that the vector contains one or several copies of two or more of the nucleic acid molecules of the invention whereby the two or more of the nucleic acid molecule differ at least with regard to the miRNA binding site, i.e. the nucleotide sequence of the miRNA binding site, and thus with regard to the miRNA targeted by the nucleic acid molecule(s) of the invention.

The vector of the invention preferably allows the expression of the nucleic acid molecule contained in the vector; in a preferred embodiment the expression results in the production of transcripts of the nucleic acid molecule of the invention and, respectively, of nucleic acid molecules of the invention.

The vector of the invention may be an RNA or DNA vector containing the nucleic acid molecule of the invention. The vector of the invention may be a plasmid containing the nucleic acid molecule of the invention, an episomal vector such as pEPI containing the nucleic acid molecule of the invention, a plasmid including but not limited to a bacterial artificial chromosome containing the nucleic acid molecule of the invention or a viral vector containing the nucleic acid molecule of the invention. As long as a vector allows transcription, preferably an efficient transcription, of the nucleic acid molecule of the invention contained in such vector, such vector is suitable for the practicing of the present invention.

In an embodiment the vector is a viral vector. The viral vector may comprise the entire viral nucleic acid or a part thereof, whereby the part of the viral nucleic acid must still be capable of allowing transcription, preferably an efficient transcription, of the nucleic acid molecule of the invention contained in such part of the entire viral nucleic acid, i.e. vector upon introduction of such vector into a cell which, in principle, allows the transcription of the nucleic acid molecule of the invention. The part of the viral vector containing the nucleic acid molecule of the invention which is different from the nucleic acid molecule of the invention, is also referred to as the vector backbone.

It will be acknowledged by a person skilled in the art that if the nucleic acid molecule of the invention is contained in or is part of a vector, depending on the vector, the nucleic acid molecule of the invention may be either a DNA or an RNA. It will also be acknowledged by a person skilled in the art that the transcript of the nucleic acid of the invention provided by the vector of the invention is preferably an RNA.

An aspect of the invention is a vector library comprising a plurality of vectors forming the elements of the library, wherein each vector of said vector library is a vector according to the invention, wherein the vectors of the nucleic acid library differ in the sequence of the miRNA binding site. It will be acknowledged by a person skilled in the art that each vector being an element of the vector library may be present as a single copy or as several copies.

A further aspect of the invention is a cell comprising at least one nucleic acid molecule of the invention and/or at least one vector of the invention. In an embodiment the cells is a mammalian cell such as a human or primate cell, an avian cells, a vertebrate cell or an insect cell. In an embodiment, the cell is a cell selected from the group comprising fibroblast, endothelial cells, epithelial cells, stroma cells and immune cells. In a preferred embodiment of the invention the cell of the invention is a cell which is used for and/or capable of producing an agent. Such agent may be, but is not limited to a protein, preferably a therapeutically effective protein such as a hormone, enzyme, cytokine or antibody, viruses, primary metabolites such as amino acids, sugars, nucleotides and lipids, and secondary metabolites. As preferably used herein the term protein also encompasses peptides and polypeptides as known in the art.

In the embodiment of the cell of the invention and, respectively, of the nucleic acid molecule of the invention the targeted miRNA is an antiviral miRNA. Embodiments of such antiviral miRNAs are hsa-miR-24, hsa-miR-103, hsa-miR-199a-3p, and hsa-miR-214. Embodiment of such proviral miRNAs are hsa-miR-122, hsa-miR-30b, hsa-miR-30d, and hsa-miR-93. For both cases see: Proc Natl Acad Sci USA. 2010 Aug 3;107(31):13830-5. Epub 2010 Jul 19. Combined agonist-antagonist genome-wide functional screening identifies broadly active antiviral microRNAs. Santhakumar D, Forster T, Laqtom NN, Fragkoudis R, Dickinson P, Abreu-Goodger C, Manakov SA, Choudhury NR, Griffiths SJ, Vermeulen A, Enright AJ, Dutia B, Kohl A, Ghazal P, Buck AH; and Modulation of hepatitis C virus RNA abundance by a liver-specific MicroRNA. Jopling CL, Yi M, Lancaster AM, Lemon SM, Sarnow P. Science. 2005 Sep 2;309(5740):1577-81).

As to the various methods of the present invention it is to be acknowledged that the respective cell is transfected or transformed by means and procedures known by a person skilled in the art. Also, to the extent that any changes and/or effects are to be detected or determined in connection with the methods of the invention, typically any changes and/or effects of the transfected or transformed cell, such changes and/or effects can be detected or determined by a person skilled in the art by means and procedures known by a person skilled in the art.

As preferably used herein, the term modulating the activity encompasses that the respective activity can be either increased or decreased.

In a further aspect the present invention is related to a pharmaceutical composition comprising a nucleic acid molecule of the invention, a vector of the invention and/or a cell of the invention. The preparation of pharmaceutical compositions of the invention is within the routine skills of a person of the art in the light of the disclosure of the instant application.

It will be understood by a person skilled in the art that various disease may be addressed by the therapeutic aspects of the instant invention such as the methods of treatment and the pharmaceutical composition disclosed herein. All of them are based on the concept that the nucleic acid molecule is specifically and effectively inhibiting and preferably degrading the miRNA targeted by the nucleic acid molecule of the invention. Because of this the nucleic acid molecule and any vector, cell or composition comprising the same, can be used in the treatment of any disease or condition which involves the targeted miRNA, i.e. any disease or disorder where inhibiting the effect or functioning of said targeted miRNA is suitable to treat such disease or condition or to ameliorate the symptoms of such disease and disorder.

The various SEQ ID NOs., the actual sequence thereof and the internal reference number are summarized in the following table.

**Table 1: Sequences of the present invention**

| **SEQ ID No.** | **Sequence** | **internal reference** |
|---|---|---|
| 1 | AGAAGUCCCUUCGGAUGUCUGCCUGA GAACGGUCUCCUCAUCGGGAGAUG | 50 nt 3' flanking nucleotide sequence |
| 2 | AUCCUCGUCCGCCGUCUCCCCCCGUCC GUGAUCUCCCCCAGACGACGAUC | 50 nt 5' flanking nucleotide sequence |
| 3 | AGAAGUCCCUUCGGAUGUCUGCCUGA GAACGGUCUCCUCAUCGGGAGAUGCU ACUUGAAUGCAAUACGCUUUUGUUU UUUACGAGAACGUGUUCUUUCACGCA CAUACAUCCUUCCCUCGUGUGUCCGU CCUCGCAUGCUCCCUAGUUCGUAGAU GACCCGUCCUUAGGUUUCGUUUCUGU GUAAACGGCUCGUGAAGUUCGUCUU CUCGGUCGCCCCGCUAUAGCUCCGUCC GAGACCCUCAUCGUGGUUCCCUAUAU GUUCUAAGACCUCCGGGUCGCGGUGC UAACAUGCCACUAAAUCCUCACCGUC AACCGUCGGCUGGUCCGAUCGGUCGA CGGCCGUCCGUCUUCCGUGUUAUCCA CUUAGCAUUAAGAUCCCUGACCUGUC CCGACCUUUUUCCUCGUAACCUUGAC CGUUCCAUGACCCCAGCUCCCGAAUG ACCUUUUGUCCUUCCCCGAUGUUUUU CCCCUCGCCCUUCGUCACAUCGAACGG UCAGUUCGUUCCCGGGGUUGUUUUC UGUUCCGAGCGGACGUCGGGGGGUCG GUCUCAUCCGGGACGACCUCUCAUCU CCUUCCCGCUUGAGCUUUCGCCAGUU CGAAUCAAUAAAAGCUAUUCAAUGAU UUGACAUCUCGUCUGGUACAUCGUGU UUUUCUGGGUGCUUACCCGCGUCACA UGGAGGGGUGGGUUC | 693 nt 3' flanking nucleotide sequence; polyadenylation-(polyA)-signal indicated in bold and underlined |
| 4 | AACAUCCGACCGACUGCCCGUCUCCU UCGGAGUAGAAACGCUAUCCUCGGAG CGAUCCUCGUCCGCCGUCUCCCCCGU CCGUGAUCUCCCCCAGACGACGAUC | 104 nt 5' flanking nucleotide sequence |
| 5 | GUAGGUGCCCCCGUGAUGAGGGAGG CCCGACGCUAGAUCUGGCUCGGAUGA UAAUUAUUAACGUUGUUUUGUCUGA CAG | non-translated transfer nucleotide sequence |
| 6 | GCUCGCUGGUCGAGCGACCGCCCGGG UUUCGAGAUGCCCGUCGUAACGCGAC GCCGGUGACGCAACGCGACCAGCGUG CCGCGUCGCGGUUCGCGCUCGACCU UCGAGCGUUCCUCCGCUGUUCGAGUG UUGCCACGUACCGCGCUCCGGAGAAC GUCGUUCACGGAGGUGUUACGCGGAG CGCAUGACUGCCGGGCGAGGGCGCCC CGCCGGCAUGCUACAGGCGUUACGGG UUGACCGCCGCGCAGGUACCAGGUAA UAACGAUGUUCGUGUGUUCUGUCUUU CCAGCCGAGUUCGUGAUCGGUCAACU UUCCCUGUGAGCGCUGCGGGAGGCCG AGUGCGCGAUCUCGCUCCGCGCGCUC CCUCGCCGUUCGUGUCGCGGAUCGCU GUCCGUUCCCGCUCAUCGGUCUGACU UCUCGGAAAUCCCCGCUCUUUUGUCU CCUCCCCCGCGCGGGUGCGCUUGAAG UGUAUAAAAGCUGAGUGUGGAGCGGU CGCAGU | 500 nt m169 promoter; TATA-Box indicated in bold and underlined |
| 7 | GGGCGCCCCGCCGGCAUGCUACAGGC GUUACGGGUUGACCGCCGCGCAGGUA CCAGGUAAUAACGAUGUUCGUGUGUU CUGUCUUUCCAGCCGAGUUCGUGAUC GGUCAACUUUCCCUGUGAGCGCUGCG GGAGGCCGAGUGCGCGAUCUCGCUCC GCGCGCUCCCUCGCCGUUCGUGUCGC GGAUCGCUGUCCGUUCCCGCUCAUCG GUCUGACUUCUCGGAAAUCCCCGCUC UUUUGUCUCCUCCCCCGCGCGGGUGC GCUUGAAGUGUAUAAAGCUGAGUGU GGAGCGGUCGCAGU | 300 nt m169 promoter; TATA-Box indicated in bold and underlined |
| 8 | GCUCGCUGGUCGAGCGACCGCCCGGG UUUCGAGAUGCCCGUCGUAACGCGAC GCCGGUGACGCAACGCGACCAGCGUG CCGCGUCGCGGGUUCGCGCUCGACCU UCGAGCGUUCCUCCGCUGUUCGAGUG UUGCCACGUACCGCGCUCCGGAGAAC GUCGUUCACGGAGGUGUUACGCGGAG CGCAUGACUGCCGGGCGAGGGCGCCC CGCCGGCAUGCUACAGGCGUUACGGG UUGACCGCCGCGCAGGUACCAGGUAA | nucleotide sequence of the nucleic acid molecule of the invention with the targeted miRNA being miR-27a |
| | UAACGAUGUUCGUGUGUUCUGUCUUU CCAGCCGAGUUCGUGAUCGGUCAACU UUCCCUGUGAGCGCUGCGGAGGCCG AGUGCGCGAUCUCGCUCCGCGCGCUC CCUCGCCGUUCGUGUCGCGGAUCGCU GUCCGUUCCCGCUCAUCGGUCUGACU UCUCGGAAAUCCCCGCUCUUUGUCU CCUCCCCCGCGCGGGUGCGCUUGAAG UGUAUAAAGCUGAGUGUGGAGCGGU CGCAGUCGACAGCUUCACAUCCCGGU GACCUGUCGCUUUCGGUGGUCUCUCU CUGCCUGUCUGUGGUGAGCUGUGCUC CGGCGGUUCCCGCGUCCGUCAUGAGG UCCAGGUCGUGUGACACGCUAAAUAU GGCCAUCGAUAACGGUGCAUGUAUCA CACAAACCCUCCGUUCCAGUGAACAC UGCCUGCAUACCUCUGUCGCUAACGC CGAAGAAGAUUUCUGCGAAAAUCAGC UGCUGCGUCGUCUGGCCCACACCGUG UGCAGUGGAAUGACCAUGCGUAAGAC CGAAACCAAGUCCUGCUGGUCUGUCA GCUUCGCGCGCGACGAAGUUGCGAUG GAUACGUGUAUCAACACCAUCUAAUC AGCUUUUCGCUCCCCGCGACGCCGGA CUCGCGAUGAGCAACGCGGUCCCGUU CGUCCCCUACGCGGGUAUCGGCCACA ACCUUCGUCGCUGGGAGCUGCAAUGU CGUCCGACCGACACCGAGCUUGCUAC UUUCUCCGCCACUUCAUCGUUCAUGU CACCGAAAGUGUUCGGUGACGAUAGC CGUUGCCGUCUUUUCUGUUGCUGUCC CUACGAUCCCCGCGAACCCUACGGCC UCACCCGCUGGGUGUCGCAUUCUACC UCCCUGACCUGGAUUCACGUCGCAUC GCCGUGUACUACACGAACGUCCGUCC GAUCAGCGAGCGUCCUCUGUUCGCCC AGCGCGGUUGGAGACGUCGUUAUCAG ACGUUCGCCAAGAUCUUGGAUACGCA GGAGCGGCGGGCCACCGAAAGAGGUA GGUGCCCCCCGUGAUGAGGGAGGCCC GACGCUAGAUCUGGCUCGGAUGAUAA UUAUUAACGUUGUUUUUGUCUGACAG GUGACAACCUCUCCGGCUGGCUCUCG GUCCUCGACGUGAAUCCUCCUUCCCC UCCGUCCUGAAACAUCCGACCGACUG CCCGUCUCCUUCGGAGUAGAAACGCU AUCCUCGGAGCGAUCCUCGUCCGCCG UCUCCCCCCGUCCGUGAUCUCCCCCAG | |
| | ACGACGAUCUGCGGAAUAAUAAGCUG UGAAAAGAAGUCCCUUCGGAUGUCUG CCUGAGAACGGUCUCCUCAUCGGGAG AUGCUACUUGAAUGCAAUACGCUUUU UGUUUUUACGAGAACGUGUUCUUUC ACGCACAUACAUCCUUCCCUCGUGUG UCCGUCCUCGCAUGCUCCCUAGUUCG UAGAUGACCCGUCCUUAGGUUUCGUU UCUGUGUAAACGGCUCGUGAAGUUUC GUCUUCUCGGUCGCCCCGCUAUAGCU CCGUCCGAGACCCUCAUCGUGGUUCC CUAUAUGUUCUAAGACCUCCGGGUCG CGGUGCUAACAUGCCACUAAAUCCUC ACCGUCAACCGUCGGCUGGUCCGAUC GGUCGACGGCCGUCCGUCUUCCGUGU UAUCCACUUAGCAUUAAGAUCCCUGA CCUGUCCCGACCUUUUUCCUCGUAAC CUUGACCGUUCCAUGACCCCAGCUCCC GAAUGACCUUUUGUCCUUCCCCGAUG UUUUUCCCCUCGCCCUUCGUCACAUC GAACGGUCAGUUCGUUCCCGGGGUUG UUUUUCUGUUCCGAGCGGACGUCGGG GGGUCGGUCUCAUCCGGGACGACCUC UCAUCUCCUUCCCGCUUGAGCUUUCG CCAGUUCGAAUCAAUAAAAGCUAUUC AAUGAUUUGACAUCUCGUCUGGUACA UCGUGUUUUUCUGGGUGCUUACCCGC GUCACAUGGAGGGGUGGGUUC | |
| 9 | GCUCGCUGGUCGAGCGACCGCCCGGG UUUCGAGAUGCCCGUCGUAACGCGAC GCCGGUGACGCAACGCGACCAGCGUG CCGCGUCGCGGGUUCGCGCUCGACCU UCGAGCGUUCCUCCGCUGUUCGAGUG UUGCCACGUACCGCGCUCCGGAGAAC GUCGUUCACGGAGGUGUUACGCGGAG CGCAUGACUGCCGGGCGAGGGCGCCC CGCCGGCAUGCUACAGGCGUUACGGG UUGACCGCCGCGCAGGUACCAGGUAA UAACGAUGUUCGUGUGUUCUGUCUUU CCAGCCGAGUUCGUGAUCGGUCAACU UUCCCUGUGAGCGCUGCGGGAGGCCG AGUGCGCGAUCUCGCUCCGCGCGCUC CCUCGCCGUUCGUGUCGCGGAUCGCU GUCCGUUCCCGCUCAUCGGUCUGACU UCUCGGAAAUCCCCGCUCUUUGUCU CCUCCCCCGCGCGGGUGCGCUUGAAG UGUAUAAAAGCUGAGUGUGGAGCGGU | nucleotide sequence attached to the 5' end of the miRNA binding site |
| | CGCAGUCGACAGCUUCACAUCCCGGU GACCUGUCGCUUUCGGUGGUCUCUCU CUGCCUGUCUGUGGUGAGCUGUGCUC CGGCGGUUCCCGCGUCCGUCAUGAGG UCCAGGUCGUGUGACACGCUAAAUAU GGCCAUCGAUAACGGUGCAUGUAUCA CACAAACCCUCCGUUCCAGUGAACAC UGCCUGCAUACCUCUGUCGCUAACGC CGAAGAAGAUUUCUGCGAAAAUCAGC UGCUGCGUCGUCUGGCCCACACCGUG UGCAGUGGAAUGACCAUGCGUAAGAC CGAAACCAAGUCCUGCUGGUCUGUCA GCUUCGCGCGCGACGAAGUUGCGAUG GAUACGUGUAUCAACACCAUCUAAUC AGCUUUUCGCUCCCCGCGACGCCGGA CUCGCGAUGAGCAACGCGGUCCCGUU CGUCCCCUACGCGGGUAUCGGCCACA ACCUUCGUCGCUGGGAGCUGCAAUGU CGUCCGACCGACACCGAGCUUGCUAC UUUCUCCGCCACUUCAUCGUUCAUGU CACCGAAAGUGUUCGGUGACGAUAGC CGUUGCCGUCUUUUCUGUUGCUGUCC CUACGAUCCCCGCGAACCCUACGGGCC UCACCCGCUGGGUGUCGCAUUCUACC UCCCUGACCUGGAUUCACGUCGCAUC GCCGUGUACUACACGAACGUCCGUCC GAUCAGCGAGCGUCCUCUGUUCGCCC AGCGCGGUUGGAGACGUCGUUAUCAG ACGUUCGCCAAGAUCUUGGAUACGCA GGAGCGGCGGGCCACCGAAAGAGGUA GGUGCCCCCCGUGAUGAGGGAGGCCC GACGCUAGAUCUGGCUCGGAUGAUAA UUAUUAACGUUGUUUUGUCUGACAG GUGACAACCUCUCCGGCUGGCUCUCG GUCCUCGACGUGAAUCCUCCUUCCCC UCCGUCCUGAAACAUCCGACCGACUG CCCGUCUCCUUCGGAGUAGAAACGCU AUCCUCGGAGCGAUCCUCGUCCGCCG UCUCCCCCCGUCCGUGAUCUCCCCCAG ACGACGAUC | |
| 10 | AGAAGUCCCUUCGGAUGUCUGCCUGA GAACGGUCUCCUCAUCGGGAGAUGCU ACUUGAAUGCAAUACGCUUUUGUUU UUUACGAGAACGUGUUCUUCACGCA CAUACAUCCUUCCCUCGUGUGUCCGU CCUCGCAUGCUCCCUAGUUCGUAGAU GACCCGUCCUUAGGUUCGUUUCUGU | nucleotide sequence attached to the 3' end of the miRNA binding site |
| | GUAAACGGCUCGUGAAGUUUCGUCUU CUCGGUCGCCCCGCUAUAGCUCCGUCC GAGACCCUCAUCGUGGUUCCCUAUAU GUUCUAAGACCUCCGGGUCGCGGUGC UAACAUGCCACUAAAUCCUCACCGUC AACCGUCGGCUGGUCCGAUCGGUCGA CGGCCGUCCGUCUUCCGUGUUAUCCA CUUAGCAUUAAGAUCCCUGACCUGUC CCGACCUUUUUCCUCGUAACCUUGAC CGUUCCAUGACCCCAGCUCCCGAAUG ACCUUUUGUCCUUCCCCGAUGUUUUU CCCCUCGCCCUUCGUCACAUCGAACGG UCAGUUCGUUCCCGGGGUUGUUUUC UGUUCCGAGCGGACGUCGGGGGUCG GUCUCAUCCGGGACGACCUCUCAUCU CCUUCCCGCUUGAGCUUUCGCCAGUU CGAAUCAAUAAAAGCUAUUCAAUGAU UUGACAUCUCGUCUGGUACAUCGUGU UUUUCUGGGUGCUUACCCGCGUCACA UGGAGGGGUGGGUUC | |
| 11 | AUGAGCAACGCGGUCCCGUUCGUCCC CUACGCGGGUAUCGGCCACAACCUUC GUCGCUGGGAGCUGCAAUGUCGUCCG ACCGACACCGAGCUUGCUACUUUCUC CGCCACUUCAUCGUUCAUGUCACCGA AAGUGUUCGGUGACGAUAGCCGUUGC CGUCUUUUCUGUUGCUGUCCCUACGA UCCCCGCGAACCCUACGGGCCUCACCC GCUGGGUGUCGCAUUCUACCUCCCUG ACCUGGAUUCACGUCGCAUCGCCGUG UACUACACGAACGUCCGUCCGAUCAG CGAGCGUCCUCUGUUCGCCCAGCGCG GUUGGAGACGUCGUUAUCAGACGUUC GCCAAGAUCUUGGAUACGCAGGAGCG GCGGGCCACCGAAAGAGGUAGGUGCC CCCCGUGAUGAGGGAGGCCCGACGC UAGAUCUGGCUCGGAUGAUAAUUAU UAACGUUGUUUUGUCUGACAGGUG ACAACCUCUCCGGCUGGCUCUCGGUC CUCGACGUGAAUCCUCCUUCCCCUCCG UCCUGA | nucleotide sequence coding for m169, wherein a non-translated transfer nucleotide sequence of 81 nt indicated in bold and underlined is contained within the coding sequence; stop codon indicated in bold and italic |
| 12 | AUGGUGAGCAAGGGCGAGGAGCUGUU CACCGGGGUGGUGCCCAUCCUGGUCG AGCUGGACGGCGACGUAAACGGCCAC AAGUUCAGCGUGUCCGGCGAGGGCGA | EGFP coding sequence |
| | GGGCGAUGCCACCUACGGCAAGCUGA CCCUGAAGUUCAUCUGCACCACCGGC AAGCUGCCCGUGCCCUGGCCCACCCUC GUGACCACCCUGACCUACGGCGUGCA GUGCUUCAGCCGCUACCCCGACCACA UGAAGCAGCACGACUUCUUCAAGUCC GCCAUGCCCGAAGGCUACGUCCAGGA GCGCACCAUCUUCUUCAAGGACGACG GCAACUACAAGACCCGCGCCGAGGUG AAGUUCGAGGGCGACACCCUGGUGAA CCGCAUCGAGCUGAAGGGCAUCGACU UCAAGGAGGACGGCAACAUCCUGGGG CACAAGCUGGAGUACAACUACAACAG CCACAACGUCUAUAUCAUGGCCGACA AGCAGAAGAACGGCAUCAAGGUGAAC UUCAAGAUCCGCCACAACAUCGAGGA CGGCAGCGUGCAGCUCGCCGACCACU ACCAGCAGAACACCCCCAUCGGCGAC GGCCCCGUGCUGCUGCCCGACAACCAC UACCUGAGCACCCAGUCCGCCCUGAG CAAAGACCCCAACGAGAAGCGCGAUC ACAUGGUCCUGCUGGAGUUCGUGACC GCCGCCGGGAUCACUCUCGGCAUGGA CGAGCUGUACAAGUAA | |
| 13 | AUGAGCAACGCGGUCCCGUUCGUCCC CUACGCGGUAUCGGCCACAACCUUC GUCGCUGGGAGCUGCAAUGUCGUCCG ACCGACACCGAGCUUGCUACUUUCUC CGCCACUUCAUCGUUCAUGUCACCGA AAGUGUUCGGUGACGAUAGCCGUUGC CGUCUUUUCUGUUGCUGUCCCUACGA UCCCCGCGAACCCUACGGCCUCACCC GCUGGGUGUCGCAUUCUACCUCCCUG ACCUGGAUUCACGUCGCAUCGCCGUG UACUACACGAACGUCCGUCCGAUCAG CGAGCGUCCUCUGUUCGCCCAGCGCG GUUGGAGACGUCGUUAUCAGACGUUC GCCAAGAUCUUGGAUACGCAGGAGCG GCGGGCCACCGAAAGAGGUGACAACC UCUCCGGCUGGCUCUCGGUCCUCGAC GUGAAUCCUCCUUCCCCUCCGUCCUG A | m169 coding sequence without non-translated transfer nucleotide sequence; stop codon indicated in bold and underlined |
| 14 | AUGGCCGAUGCUAAGAACAUUAAGAA GGGCCCUGCUCCCUUCUACCCUCUGG AGGAUGGCACCGCUGGCGAGCAGCUG CACAAGGCCAUGAAGAGGUAUGCCCU GGUGCCUGGCACCAUUGCCUUCACCG | Firefly Luciferase coding sequence |
| | AUGCCCACAUUGAGGUGGACAUCACC UAUGCCGAGUACUUCGAGAUGUCUGU GCGCCUGGCCGAGGCCAUGAAGAGGU ACGGCCUGAACACCAACCACCGCAUC GUGGUGUGCUCUGAGAACUCUCUGCA GUUCUUCAUGCCAGUGCUGGGCGCCC UGUUCAUCGGAGUGGCCGUGGCCCCU GCUAACGACAUUUACAACGAGCGCGA GCUGCUGAACAGCAUGGGCAUUUCUC AGCCUACCGUGGUGUUCGUGUCUAAG AAGGGCCUGCAGAAGAUCCUGAACGU GCAGAAGAAGCUGCCUAUCAUCCAGA AGAUCAUCAUCAUGGACUCUAAGACC GACUACCAGGGCUUCCAGAGCAUGUA CACAUUCGUGACAUCUCAUCUGCCUC CUGGCUUCAACGAGUACGACUUCGUG CCAGAGUCUUUCGACAGGGACAAAAC CAUUGCCCUGAUCAUGAACAGCUCUG GGUCUACCGGCCUGCCUAAGGGCGUG GCCCUGCCUCAUCGCACCGCCUGUGU GCGCUUCUCUCACGCCCGCGACCCUAU UUUCGGCAACCAGAUCAUCCCCGACA CCGCUAUUCUGAGCGUGGUGCCAUUC CACCACGGCUUCGGCAUGUUCACCAC CCUGGGCUACCUGAUUUGCGGCUUUC GGGUGGUGCUGAUGUACCGCUUCGAG GAGGAGCUGUUCCUGCGCAGCCUGCA AGACUACAAAAUUCAGUCUGCCCUGC UGGUGCCAACCCUGUUCAGCUUCUUC GCUAAGAGCACCCUGAUCGACAAGUA CGACCUGUCUAACCUGCACGAGAUUG CCUCUGGCGGCGCCCCACUGUCUAAG GAGGUGGGCGAAGCCGUGGCCAAGCG CUUUCAUCUGCCAGGCAUCCGCCAGG GCUACGGCCUGACCGAGACAACCAGC GCCAUUCUGAUUACCCCAGAGGGCGA CGACAAGCCUGGCGCCGUGGGCAAGG UGGUGCCAUUCUUCGAGGCCAAGGUG GUGGACCUGGACACCGGCAAGACCCU GGGAGUGAACCAGCGCGGCGAGCUGU GUGUGCGCGGCCCUAUGAUUAUGUCC GGCUACGUGAAUAACCCUGAGGCCAC AAACGCCCUGAUCGACAAGGACGGCU GGCUGCACUCUGGCGACAUUGCCUAC UGGGACGAGGACGAGCACUUCUUCAU CGUGGACCGCCUGAAGUCUCUGAUCA AGUACAAGGGCUACCAGGUGGCCCCA GCCGAGCUGGAGUCUAUCCUGCUGCA | |
| | GCACCCUAACAUUUUCGACGCCGGAG UGGCCGGCCUGCCCGACGACGAUGCC GGCGAGCUGCCUGCCGCCGUCGUCGU GCUGGAACACGGCAAGACCAUGACCG AGAAGGAGAUCGUGGACUAUGUGGCC AGCCAGGUGACAACCGCCAAGAAGCU GCGCGGCGGAGUGGUGUUCGUGGACG AGGUGCCCAAGGGCCUGACCGGCAAG CUGGACGCCCGCAAGAUCCGCGAGAU CCUGAUCAAGGCUAAGAAAGGCGGCA AGAUCGCCGUGUAA | |
| 15 | CGACAGCUUCACAUCCCGGUGACCUG UCGCUUUCGGUGGUCUCUCUCUGCCU GUCUGUGGUGAGCUGUGCUCCGGCGG UUCCCGCGUCCGUCAUGAGGUCCAGG UCGUGUGACACGCUAAAUAUGGCCAU CGAUAACGGUGCAUGUAUCACACAAA CCCUCCGUUCCAGUGAACACUGCCUG CAUACCUCUGUCGCUAACGCCGAAGA AGAUUUCUGCGAAAAUCAGCUGCUGC GUCGUCUGGCCCACACCGUGUGCAGU GGAAUGACCAUGCGUAAGACCGAAAC CAAGUCCUGCUGGUCUGUCAGCUUCG CGCGCGACGAAGUUGCGAUGGAUACG UGUAUCAACACCAUCUAAUCAGCUUU UCGCUCCCCGCGACGCCGGACUCGCGA UGAGCAACGCGGUCCCGUUCGUCCCC UACGCGGGUAUCGGCCACAACCUUCG UCGCUGGGAGCUGCAAUGUCGUCCGA CCGACACCGAGCUUGCUACUUUCUCC GCCACUUCAUCGUUCAUGUCACCGAA AGUGUUCGGUGACGAUAGCCGUUGCC GUCUUUUCUGUUGCUGUCCCUACGAU CCCCGCGAACCCUACGGGCCUCACCCG CUGGGUGUCGCAUUCUACCUCCCUGA CCUGGAUUCACGUCGCAUCGCCGUGU ACUACACGAACGUCCGUCCGAUCAGC GAGCGUCCUCUGUUCGCCCAGCGCGG UUGGAGACGUCGUUAUCAGACGUUCG CCAAGAUCUUGGAUACGCAGGAGCGG CGGGCCACCGAAAGAGGUAGGUGCCC CCCGUGAUGAGGGAGGCCCGACGCU AGAUCUGGCUCGGAUGAUAAUUAUU AACGUUGUUUUGUCUGACAGGUGA CAACCUCUCCGGCUGGCUCUCGGUCC UCGACGUGAAUCCUCCUUCCCCUCCG UCCUGAAACAUCCGACCGACUGCCCG contained | m169 transcript sequence wherein a non-translated transfer nucleotide sequence indicated in bold and underlined is contained within the coding sequence; miR27a binding site bold and underlined, with miR27a binding site being located downstream of the non-translated transfer nucleotide sequence |
| | UCUCCUUCGGAGUAGAAACGCUAUCC UCGGAGCGAUCCUCGUCCGCCGUCUC CCCCCGUCCGUGAUCUCCCCCAGACGA CGAUCUGCGGAAUAAUAAGCUGUGA AAAGAAGUCCCUUCGGAUGUCUGCCU GAGAACGGUCUCCUCAUCGGGAGAUG CUACUUGAAUGCAAUACGCUUUUUGU UUUUUACGAGAACGUGUUCUUUCACG CACAUACAUCCUUCCCUCGUGUGUCC GUCCUCGCAUGCUCCCUAGUUCGUAG AUGACCCGUCCUUAGGUUUCGUUUCU GUGUAAACGGCUCGUGAAGUUUCGUC UUCUCGGUCGCCCCGCUAUAGCUCCG UCCGAGACCCUCAUCGUGGUUCCCUA UAUGUUCUAAGACCUCCGGGUCGCGG UGCUAACAUGCCACUAAAUCCUCACC GUCAACCGUCGGCUGGUCCGAUCGGU CGACGGCCGUCCGUCUUCCGUGUUAU CCACUUAGCAUUAAGAUCCCUGACCU GUCCCGACCUUUUUCCUCGUAACCUU GACCGUUCCAUGACCCCAGCUCCCGA AUGACCUUUUGUCCUUCCCCGAUGUU UUUCCCCUCGCCCUUCGUCACAUCGA ACGGUCAGUUCGUUCCCGGGGUUGUU UUUCUGUUCCGAGCGGACGUCGGGGG GUCGGUCUCAUCCGGGACGACCUCUC AUCUCCUUCCCGCUUGAGCUUUCGCC AGUUCGAAUCAAUAAAAGCUAUUCAA UGAUUUGACAUCUCGUCUGGUACAUC GUGUUUUUCUGGGUGCUUACCCGCGU CACAUGGAGGGGUGGGUUC | |
| 16 | UGGAGUGUGACAAUGGUGUUUG | hsa-miR-122; Accession number: MIMAT0000421 |
| 17 | UGGCUCAGUUCAGCAGGAACAG | hsa-miR-24; Accession number: MIMAT0000080 |
| 18 | AGCAGCAUUGUACAGGGCUAUGA | hsa-miR-103; Accession number: MIMAT0000101 |
| 19 | ACAGUAGUCUGCACAUUGGUUA | hsa-miR-199a-3p; Accession number: MIMAT0000232 |
| 20 | ACAGCAGGCACAGACAGGCAGU | hsa-miR-214; Accession number: MIMAT0000271 |
| 21 | UGUAAACAUCCUACACUCAGCU | hsa-miR-30b; Accession number: MIMAT0000420 |
| 22 | UGUAAACAUCCCCGACUGGAAG | hsa-miR-30d; Accession number: MIMAT0000245 |
| 23 | CAAAGUGCUGUUCGUGCAGGUAG | hsa-miR-93; Accession number: MIMAT0000093 |
| 24 | AAGUGACGGUGAGAUCCAGGCU | hcmv-miR-UL112; Accession number: MIMAT0001577 |
| 25 | uucacaguggcuaaguucCgc | miR-27a |
| 26 | uucacaguggcuaaguucUgc | miR-27b |
| 27 | TGAGGAGACCGTTCTCAGGCAGACATC CGAAGGACTTCTTTCCATAGTTTATT ATTCCGCAGATCGTCGTCTGGGGAGA TAGGATGACGACGATAAGTAGGG | H5-m169-pepkan |
| 28 | CAACCAATTAACCAATTCTGATTAG | Pepkan-rev |
| 29 | TGAGGAGACCGTTCTCAGGCAGAC | m169-pepkan-for |
| 30 | CCTCGGAGCGATCCTCGTCCGCCGTCT CCCCCCGTCCGTGATCTCCCCCAGACG ACGATCTGCGGAATAATAAACTATGGA AAGAAGTCCCTTCGGATGTCTCAACCA ATTAACCAATTCTGATTAG | H3-m169-pepkan |
| 31 | TGAGGAGACCGTTCTCAGGCAGACATC CGAAGGGACTTCTTTAGCAGCAAACC CTATTGGCGAGATCGTCGTCTGGGGG AGATAGGATGACGACGATAAGTAGGG | H5-m169-miR16-pepkan |
| 32 | TGAGGAGACCGTTCTCAGGCAGAC | m 169-miR-16-pepkan-for |
| 33 | CCTCGGAGCGATCCTCGTCCGCCGTCT CCCCCCGTCCGTGATCTCCCCCAGACG ACGATCTCGCCAATAGGGTTTGCTGC TAAAGAAGTCCCTTCGGATGTCTCAAC CAATTAACCAATTCTGATTAG | H3-m169-miR16-pepkan |
| 34 | TGAGGAGACCGTTCTCAGGCAGACATC CGAAGGGACTTCTTATGGGGGCGTTT | H5-m169-miR-M23-2-pepkan |
| | ACAAGCGGAGATCGTCGTCTGGGGGA GATAGGATGACGACGATAAGTAGGG | |
| 35 | TGAGGAGACCGTTCTCAGGCAGAC | m169-miR-M23-2-pepkan-for |
| 36 | CCTCGGAGCGATCCTCGTCCGCCGTCT CCCCCCGTCCGTGATCTCCCCCAGACG ACGATCTCCGCTTGTAAACGCCCCCA TAAGAAGTCCCTTCGGATGTCTCAACC AATTAACCAATTCTGATTAG | H3-m 169-miR-M23-2-pepkan |
| 37 | TGAGGAGACCGTTCTCAGGCAGACATC CGAAGGGACTTCTTTTCACAGCTTATT ATTCCGCAGATCGTCGTCTGGGGGAG ATAGGATGACGACGATAAGTAGGG | H5-m169-rev-pepkan |
| 38 | TGAGGAGACCGTTCTCAGGCAGAC | m169-rev-pepkan-for |
| 39 | CCTCGGAGCGATCCTCGTCCGCCGTCT CCCCCCGTCCGTGATCTCCCCCAGACG ACGATCTGCGGAATAATAAGCTGTGA AAAAGAAGTCCCTTCGGATGTCTCAAC CAATTAACCAATTCTGATTAG | H3-m169-rev-pepkan |
| 40 | TGGCTCAGTTCAGCAGGAACAG | miR-24-for |
| 41 | TTCACAGTGGCTAAGTTCCGC | miR-27a-for |
| 42 | TAGCAGCACGTAAATATTGGCG | miR-16-for |
| 43 | ATGGGGGCCTCGGTCAAGCGGAA | mcmv-miR-M23-2-for |
| 44 | **UGCGGAAUAAUAAGCUGUGAAA** | miR27a binding site |

The present invention is now further illustrated by the following figures and examples from which further features, embodiments and advantages may be taken.

More specifically,
Fig. 1A and Fig. 1B are schematic illustrations of nucleic acid molecules according to the present invention;
Fig. 2 is a representation of a possible secondary structure of the nucleic acid molecule according to the present invention;
Fig. 3A is a schematic graph of a gene region between m167 and m170 of the MCMV genome indicating sequences deleted in various MCMV single gene deletion mutants.
Fig. 3B is a diagram showing the fold change of miR-27a levels after infection of fibroblasts with different MCMV single gene deletion mutants.
Fig. 4A is a schematic graph showing a predicted miRNA binding site and indicating binding thereof to cellular miRNA miR-27a.
Fig. 4B is a figure depicting the sequence coverage across the m167-m170 genomic locus indicating the m169 transcript as well as identifying an intron of 81 nucleotides.
Fig. 5A is a schematic graph showing the miRNA binding site of m169 and indicating binding thereof to miR-27a, as well as point mutations introduced into said miRNA binding site to abolish regulation of miR-27a
Fig. 5B is a diagram showing the fold change of miR-27a levels after infection of fibroblasts with MCMV wild-type, an MCMV mutant with point mutations introduced into miRNA binding site and a corresponding revertant MCMV mutant.
Fig. 6A are schematic graphs showing the structures and sequences of miRNA binding sites for miR-16 as well as for mcmv-miR-M23-2, said binding sites being introduced into MCMV gene m169, thereby replacing the miRNA binding site for miR-27a.
Fig. 6B is a diagram showing an extent of degradation of miR27a after infection of fibroblasts on the respective miRNA they were designed to target.
Fig. 6C is a diagram showing the fold change of miR27a, miR-16 or miR-M23-2 after infection of fibroblasts with MCMV mutants with miRNA binding sites targeting said miRNAs.

### Brief Description of the Figures

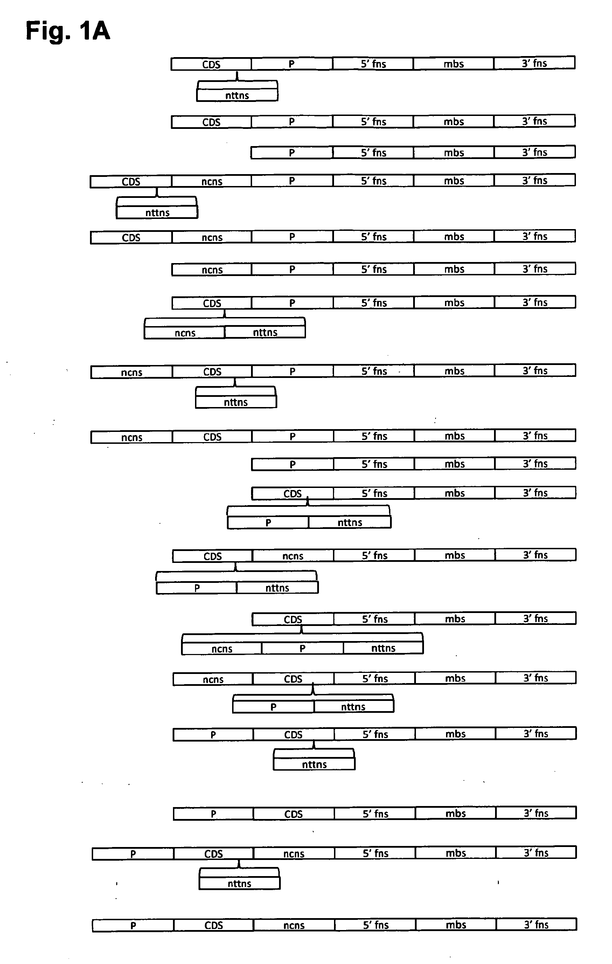

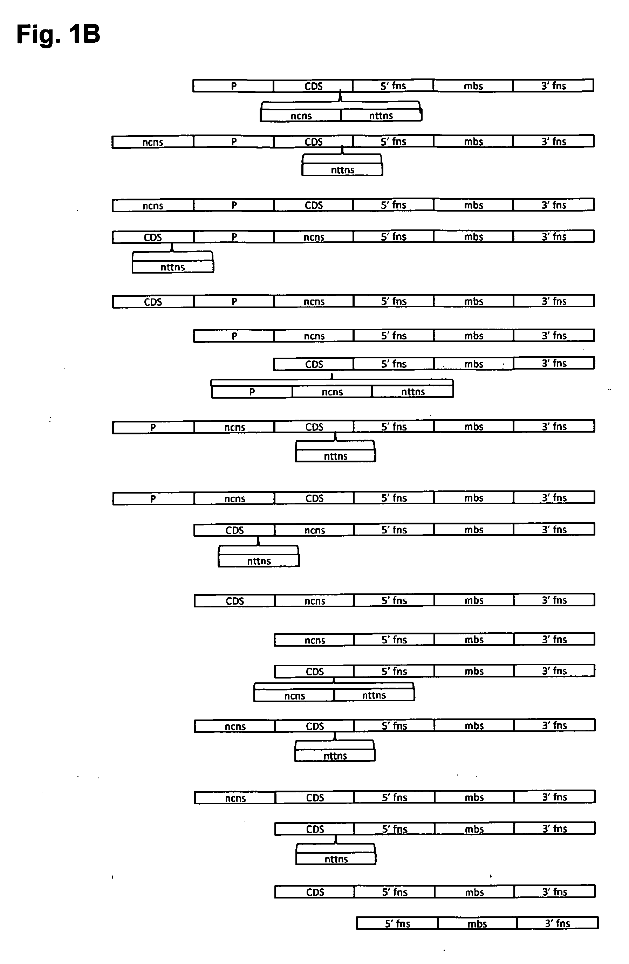

### Fig. 1A and 1B: Schematic illustration of nucleic acid molecules according to the present invention

Fig. 1A and Fig. 1B are schematic illustrations of nucleic acid molecules which are embodiments of the nucleic acid molecule according to the present invention. The figure shows sequence elements of said nucleic acid molecules according to the present invention and the position of said sequence elements in the respective nucleic acid molecule in 5' - 3' orientation. A "sequence element" means a sequence of a promoter; a sequence of an miRNA binding site; a sequence of a 5' flanking nucleotide sequence; a sequence of a 3' flanking nucleotide sequence; a sequence of a non-coding nucleotide sequence; a sequence of a coding sequence; and a sequence of a non-translated transfer nucleotide sequence, respectively, according to the present invention. Each sequence element is shown as a box. More precisely, a box designated by "P" means a sequence of a promoter; by "mbs" means a sequence of an miRNA binding site; by "5' fns" means a sequence of a 5' flanking nucleotide sequence; by "3' fns" means a sequence of a 3' flanking nucleotide sequence; by "ncns" means a sequence of a non-coding nucleotide sequence; by "CDS" means a sequence of a coding sequence; and by "nttns" means and a sequence of a non-translated transfer nucleotide sequence. A brace indicates that the respective sequence elements are contained within a coding sequence.

### Fig. 2: representation of a possible secondary structure of the nucleic acid molecule according to the present invention

Fig. 2 is a representation of a possible secondary structure of the nucleic acid molecule according to the present invention generated by M-FOLD (http://mfold.rna.albany.edu/; mfold web server: 1995-2010, Michael Zuker & Nick Markham, Rensselaer Polytechnic Institute Hosted by The RNA Institute, College of Arts and Sciences, State University of New York at Albany; Supported by the SUNY Albany Research IT Group). The miRNA binding site of the specific nucleic acid is one which is capable of binding miR-27a. As is evident from the structure various motifs are formed such as bulges or stem structures. It will be acknowledged by a person skilled in the art that for example the bulges may have a different nucleotide sequence but still providing for a bulge structure. The same is also true for the various stem structures. In said stem structures, it actually does not matter on which of the two stretches of nucleotides base pairing so as to from the stem structure the individual nucleotides are positions as long as the stem structure is still formed. In this molecule both the 5' flanking nucleotide sequence and the 3' flanking nucleotide sequence consist of 50 nucleotides and the miRNA binding site consists of 22 nucleotides.

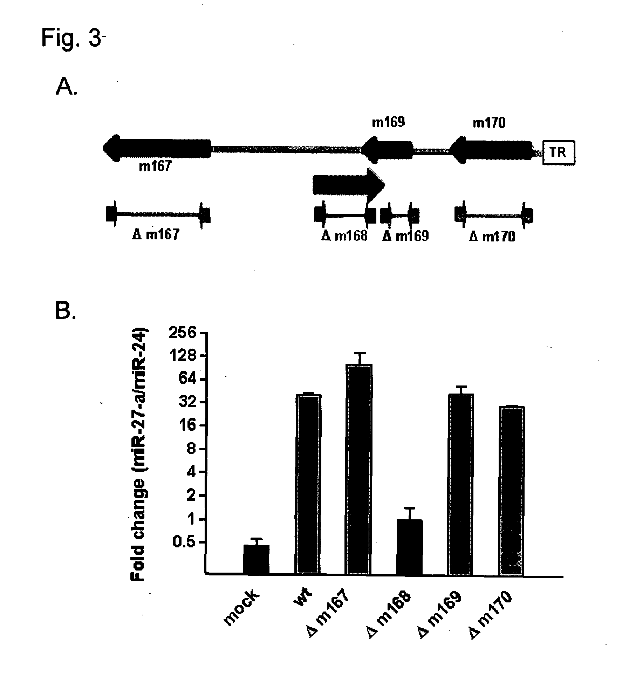

### Fig. 3A: Genomic organization of m167-m170 and single gene mutants

Fig. 3A is a schematic graph showing the genome organization of MCMV genes m167 to m170 and indicating gene fragments deleted in four MCMV deletion mutants, namely Δ167, Δ168, Δ169 and Δ170. Said MCMV deletion mutants were tested for degradation of cellular miR-27a. Dark grey arrows depict individual coding sequences, as well as the orientation of said coding sequences. The gene fragments deleted in the four MCMV mutants, namely Δ167, Δ168, Δ169 and Δ170, are indicated below the genome. Deletions indicated in light grey had no effect on miR-27a degradation while the deletion of m168 coding sequence, depicted in black, abolished the degradation of miR-27a.

### Fig. 3B: The binding site for miR-27a is located within m168

Fig. 3B is a diagram showing miR-27a levels normalized for miR-24 levels both determined by quantitative PCR 48 hours after infection of fibroblasts with MCMV mutants, namely Δ167, Δ168, Δ169 and Δ170 wherein each mutant comprises a deletion of genes as depicted in Fig.3A, wherein the effect of said deletions were tested on miR-27a. Only the mutant lacking m168 coding sequence did no longer mediate degradation of miR-27a. As controls cells were infected with MCMV wild type, also referred to herein as wt, or were left untreated, also referred to herein as "mock" or mock treatment. In the deletion mutant MCMV- Δ169 the coding region and 14 nt of the intron are deleted. Therefore, the intron is not essential, however, may still enhance miR-27a degradation. The deletion mutant MCMV-Δ169 showed that the coding sequence of m169 is not required. Therefore, the 3'UTR of m169 is sufficient to mediate degradation of miR27a.

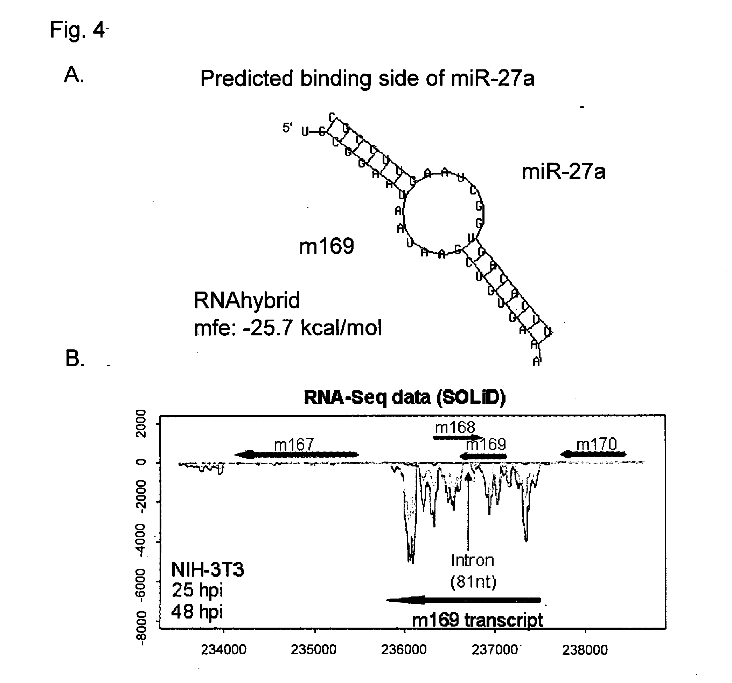

### Fig. 4A: Predicted binding site of miR-27a

Fig.4A shows a schematic graph of the binding site of the cellular targeted miR-27a and the respective miRNA binding site of m169. Said miRNA binding site has been identified being within the 3'UTR of m169 determined using RNAhybrid (http://bibiserv.techfak.uni-bielefeld.de/rnahybrid/submission.html). A free binding energy, also referred to herein as mfe, as determined by RNAhybrid is indicated in kcal/mol.

### Fig.4B Properties of the m169 transcript determined by next-generation sequencing.

Fig. 4B shows the sequence coverage of transcripts expressed from MCMV gene locus m167 to m170 as obtained by next-generation sequencing, using SOLiD system of Applied Biosystems, on total RNA prepared from MCMV wild type infected fibroblasts, 24 and 48 hours post infection, respectively. As a control total RNA prepared from uninfected cells was used. This revealed the exact sequence of the m169 transcript (SEQ ID NO:15) expressed in anti-sense orientation including its transcription start site, its sequence, an intron of 81 nucleotides, wherein the intron was identified by reads spanning across the two exons, as well as the 3'-end of the transcript. The binding site for miR-27a predicted by RNAhybrid is indicated by a "*". The x-axis indicates genomic positions within the MCMV wild type genome according to Rawlinson et al. (Rawlinson et al., supra). The y-axis depicts the coverage of the depicted sequence.

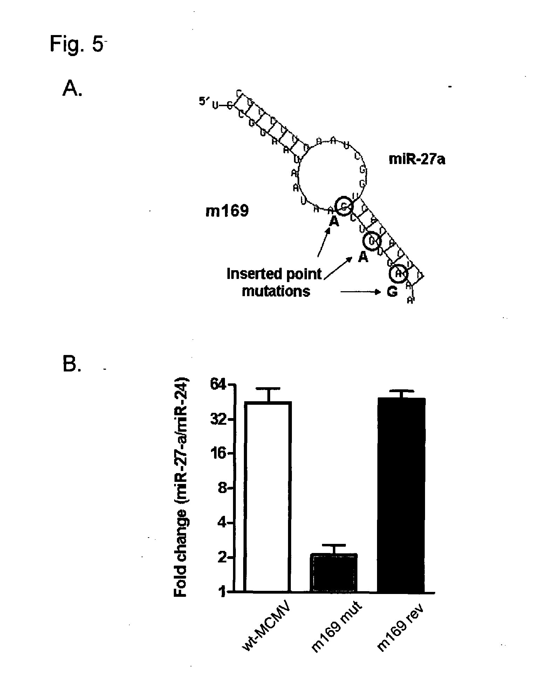

### Fig. 5A: Sequence and structure of the binding site of miR-27a, as well as point mutations introduced therein leading to loss of activity of miR-27a.

Fig. 5A shows a schematic graph depicting the binding site of miR-27a and the respective miRNA binding site of m169. Nucleotides highlighted with a circle were replaced by the respective nucleotides, indicated in bold letters, leading to MCMV-m169-mut, also referred to herein as m169-mut. The revertant-virus, i.e. m169-rev or MCMV-m169-rev, carries the wild-type sequence of the miRNA binding site of m169.

### Fig. 5B: Regulation of degradation of miR-27a is abolished in m169-mut.

Fig.5 B is a diagram showing miR-27a levels normalized for miR-24 levels both determined by quantitative PCR, 48 hours after infection of fibroblasts with MCMV mutants, namely MCMV wild type, indicated as "wt", m169-mut and m169-rev, wherein the effect of said mutants were tested on miR-27a. Degradation of miR-27a was abolished for m169-mut but restored for m169-rev.

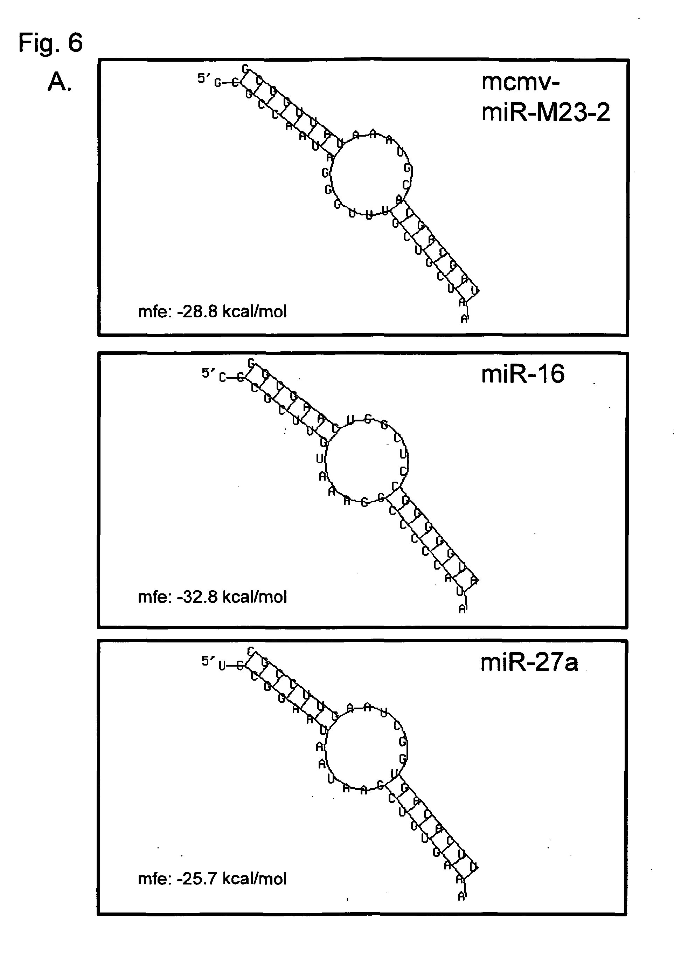

### Fig. 6A: Retargeting of m169 to other cellular and viral miRNAs by target site replacement.

Fig. 6A shows schematic graphs depicting the structure and sequence of binding sites for the cellular microRNA miR-27a and the respective target site of m169; miR-16 and the respective target site of m169-miR16; and the viral microRNA mcmv-miR-23-2 and the respective target site of m169-M23-2, depicted from the left to the right, respectively. m169-miR16, also referred to herein as MCMV-m169-miR16 and m169-M23-2, also referred to herein as MCMV-m169-M23-2 have been generated by replacing the miRNA binding site for miR-27a by a miRNA binding site for miR-16 or mcmv-miR-M23-2, respectively. Free binding energies, also referred to herein as mfe, as determined by RNAhybrid, are indicated in kcal/mol.

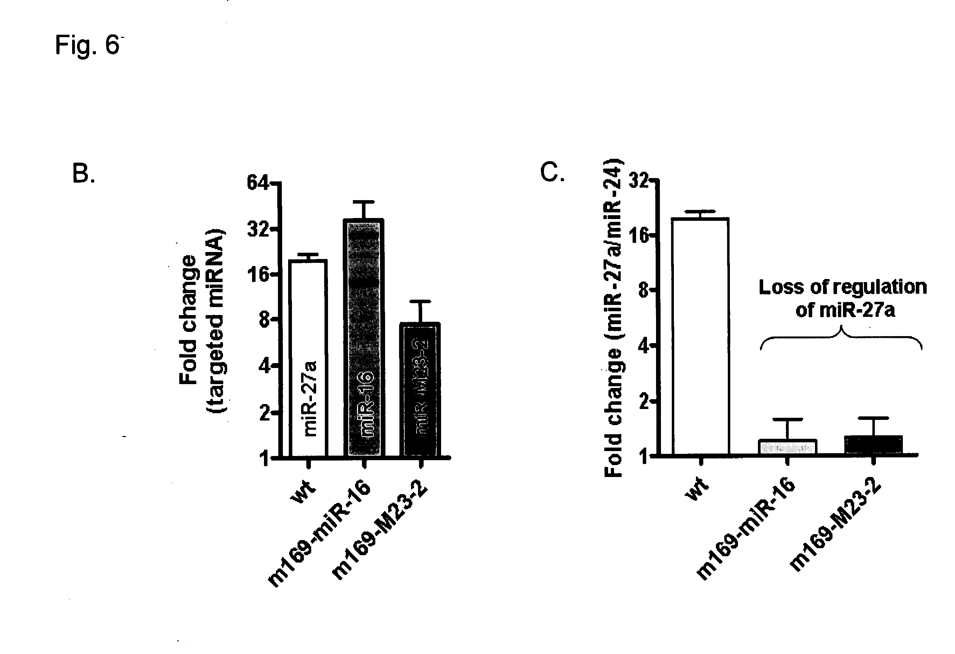

### Fig. 6B: Effect of re-targeted mutants on respective miRNA

Fig.6B is a diagram showing miR-27a, miR-16 or mcmv-miR-M23-2 levels normalized for miR-24 levels, respectively, determined by quantitative PCR, 48 hours after infection of fibroblasts with MCMV wild type, indicated as "wt", and MCMV mutants, namely m169-miR-16 and m169-M23-2, respectively, wherein the effect of said mutants was tested on the respective targeted miRNA, namely miR-27a, miR-16 and miR-M23-2, as indicated.

### Fig. 6C: Retargeted MCMV mutants no longer destabilize miR27a.

Fig. 6C is a diagram showing miR-27a levels normalized for miR-24 levels determined by quantitative PCR, 48 hours after infection of fibroblasts with MCMV wild type, depicted as "wt", and MCMV mutants, namely m169-miR-16 and m169-M23-2, wherein the effects of said mutants were tested on miR-27a. In contrast to wild-type MCMV, both m169-miR16 and m169-miR-M23-2 are no longer able to destabilize miR-27a as the miRNA binding site was changed to either the miRNA binding site for cellular miR-16 or the miRNA binding site for viral mcmv-miR-M23-2, respectively.

### Examples

### Example 1: Methods for virus mutagenesis and miRNA quantification

For quantifying levels of miRNA the following equipment and protocol was used.

NIH-3T3 cells (ATCC, CRL-1658) were grown in DMEM, also referred to herein as Dulbecco's Modified Eagle's Medium purchased from Gibco, Invitrogen, supplemented with 10% fetal bovine serum, purchased from PAN Biotech GmbH, and penicillin/streptomycin, purchased from Gibco, Invitrogen.

MCMV wild-type virus was derived from the MCMV bacterial artificial chromosome , also referred to herein as BAC, clone pSM3fr, originated from Smith strain as described in Messerle et al. (Messerle et al., Cloning and mutagenesis of a herpesvirus genome as an infectious bacterial artificial chromosome,Proc Natl Acad Sci USA. 1997 Dec 23;94(26): 14759-63.).

The m169 mutant MCMV viruses were generated by markerless BAC mutagenesis as described in Scrivano et al. (Scrivano L et al., The m74 gene product of murine cytomegalovirus (MCMV) 1 is a functional 2 homolog of human CMV gO and determines the entry pathway of MCMV (2010)).

The E.coli strain GS1783 was provided by Greg Smith, Department of Microbiology-Immunology, Ward Bldg, Northwestern University Feinberg School of Medicine, Chicago, IL 60611, and was used for electro-competent bacteria and the mutants were solely constructed in GS1783. For the mutagenesis of m169-mut an I-SceI-aphAI cassette was amplified from the plasmid pEP-KAN-S (Tischer, B. K. et al., 2006, Two-step Red13-mediated recombination for versatile high-efficiency markerless DNA manipulation in Escherichia coli. Biotechniques. 40:191-197.) by two-step touchdown-PCR using the primers H5-m169-pepkan (SEQ ID NO:27) and Pepkan-rev (SEQ ID NO:28) in the first PCR step and m169-pepkan-for (SEQ ID NO:29) and H3-m169-pepkan (SEQ ID NO:30) in the second PCR step. The primers H5-m169-pepkan and H3-m169-pepkan contain the 3 point mutations, wherein said 3 point mutations are indicated in table 1 in bold and underlined. In a first temperature induced Red recombination, the PCR fragment was inserted in pSM3fr, resulting in a BAC carrying a kanamycin resistance and an I-SceI restriction site. The kanamycin cassette was then removed from kanamycin resistant clones by an arabinose induced I-SceI digest within GS1783 and a subsequent Red recombination resulting in a markerless exchange of three nucleotides of which only the middle C → T exchange actually disrupts the binding site whereas the two flanking mutations do not, which is due to G:U and A:U base pairing. The resulting BAC was called m169-mut, leading to MCMV-m169-mut, and wherein the three point mutations are located in the seed-match of the miR-27a binding site.

Two other m169 mutant viruses were constructed by replacing the whole miR-27a binding site with a binding site for murine miR-16 or mcmv-miR-M23-2 resulting in the two MCMV mutants m169-miR-16 and m169-miR-M23-2. Cloning was performed as described for m169-mut using markerless BAC mutagenesis. In addition, the revertant of m169-mut was generated for m169-mut.

In order to generate m169-miR-16, also referred to herein as MCMV-m169-miR-16, the following primers were used: H5-m169-miR16-pepkan (SEQ ID NO:31) and Pepkan-rev (SEQ ID NO:28) for the first PCR step and m169-miR-16-pepkan-for (SEQ ID NO:32) and H3-m169-miR16-pepkan (SEQ ID NO:33) for the second PCR step. In order to generate m169-miR-M23-2, the following primers were used: H5-m169-miR-M23-2-pepkan (SEQ ID NO:34) and Pepkan-rev (SEQ ID NO: 28) were used in the first PCR step and m169-miR-M23-2-pepkan-for (SEQ ID NO:35) and H3-m169-miR-M23-2-pepkan (SEQ ID NO:36) in the second PCR step. The mutagenesis resulted in a markerless exchange of the miR-27a-binding side by a binding side for either miR-16 or miR-M23-2, which are indicated in the primer sequences depicted in table 1 in bold and underlined.

In order to generate the revertant virus m169-rev, also referred to herein as MCMV-m169-rev, the following primers were used: H5-m169-rev-pepkan (SEQ ID NO:37) and Pepkan-rev (SEQ ID NO:28) in the first PCR step and m169-rev-pepkan-for (SEQ ID NO:38) and H3-m169-rev-pepkan (SEQ ID NO:39) in the second PCR step. The mutagenesis resulted in a markerless reversion of the three point mutations back to the wild-type MCMV binding site for miR-27a, which are indicated in the primer sequences depicted in table 1 in bold and underlined.

All viruses were reconstituted by transfecting the recombinant BACs into murine embryonic fibroblasts as described in Reddehase MJ et al. (Reddehase MJ et al. (1985), interstitial murine cytomegalovirus pneumonia after irradiation: characterization of cells that limit viral replication during established infection of the lungs, J Virol 55,0264-273.). Virus titers were determined on MEFs by standard plaque assay (Reddehase et al., 1985, supra). Virus stocks were prepared on M2-10B4 cells (ATCC, CRL-1972) and viral titers were determined as described in Dölken et al. (Dölken L et al., (2007) Mouse cytomegalovirus microRNAs dominate the cellular small RNA profile during lytic infection and show features of posttranscriptional regulation. J Virol 81, 13771-13782.).

To obtain a high level of infection, centrifugal enhancement was employed during the incubation with virus as described in Dölken et al. (Dölken et al., supra). The person skilled in the art will acknowledge that said centrifugal enhancement will result in up to 10 fold higher viral load compared to incubations without said centrifugal enhancement.

In order to determine the levels of different miRNAs and their alteration following MCMV infection, NIH-3T3 cells were infected at a confluence of 80-90 % about 24 h after splitting the cells to 6-well dishes, purchased from Becton Dickinson, with the various mutants of MCMV at a multiplicity of infection, also referred to herein as MOI, of 10 thus ensuring infection of >99% of cells. Average titers of the virus stocks were in the range of 1x10⁸ plaque forming units/ml. Cells were incubated at 37 °C, 8 % CO₂. Plaque forming units is also referred to herein as PFU.

RNA was extracted at various time points according to the protocol described below. The levels of the different miRNAs, i.e. miR-27, miR-24, miR-16, mcmv-miR-M23-2 and U6, respectively, were determined by northern blot analysis or real-time quantitative PCR as described below.

### RNA preparation and northern blotting

RNA was extracted using Trizol reagent purchased from Invitrogen and Northern blotting was performed on 5 to 10 µg of total RNA as described in Dölken et al. or Pfeffer S et al. (Dölken et al., 2007, supra; Pfeffer S et al., 2005, Identification of micro RNAs of the herpesvirus family. Nat. Methods 2, 269-276), with the difference that 1-ethyl-3-(3-dimethylaminopropyl) carbodiimide (EDC)-mediated, chemical cross-linking as described in Pall et al. (Pall GS and Hamilton AJ, 2008, Improved northern blot method for enhanced detection of small RNA. Nat. Protoc 3,1077-1084), 2008) was used instead of UV cross-linking. Probes were 5' ³²P-radiolabelled oligodeoxynucleotides 100% complementary to the miRNA sequence or to part of the U6 snRNA sequence. Blots were analyzed and quantified by phosphorimaging using an FLA5100 scanner from Fuji.

### Quantitative PCR analysis of miRNAs

Quantitative real-time PCRs were established using Roche LightCycler® 480 Real-Time PCR System. For mature miRNAs, 1 µg of total RNA was poly-adenylated and reverse transcribed using the miScript Reverse Transcription kit purchased from QIAgen as per the manufacturer instructions with 1 µg of total RNA per reaction. The obtained cDNA, i.e. 1 µl of a 1:5 water dilution, was then PCR-amplified using the miScript SYBR® Green PCR kit purchased from QIAgen in 20 µl reaction mixtures consisting of Quantitect SYBR Green PCR master mix, and 0.625 µM of the miRNA specific primer and the miScript universal primer. Used Primer: miR-24-for (SEQ ID NO:40); miR-27a-for (SEQ ID NO:41); miR-16-for (SEQ ID NO:42); and mcmv-miR-M23-2-for (SEQ ID NO:43). PCR was performed using a protocol employing a hot-start enzyme activation step for 15 min at 95 °C, and 40 cycles of 95 °C denaturation for 15 s, 55 °C annealing for 30 s and 72 °C elongation for 30 s, including subsequent melting curve analysis. The data was analyzed using the ΔΔCt method as described in Bookout et al. (Bookout AL et al., 2006, High-throughput real-time quantitative reverse transcription PCR. Curr Protoc Mol Biol Chapter 15, Unit 15 18) using cellular miR-24 as an endogenous reference, and the mock infected sample as a calibrator. The amplification efficiency as determined with the LinReg software described in Ramakers et al. (Ramakers C et al., 2003, Assumption-free analysis of quantitative real-time polymerase chain reaction (PCR) data. Neurosci Lett 339, 62-66) was used to calculate fold-changes of miRNA levels in cells infected with respective viruses relative to miR-24 levels. In uninfected fibroblasts the levels of miR-24 and miR-27a were almost identical thus resulting in a miR-27a/miR-24 ratio of ~1.0. Following infection miR-24 levels remained unaltered. Therefore, this ratio resembles the extent of degradation of miR-27a by the various MCMV mutants. Primers were the mature miRNA sequences for the forward primers, and the universal miScript primer provided by QIAgen for the reverse primer.

### Example 2: Identification of miR-27a binding site in the MCMV genome

The site of the MCMV genome responsible for miR-27a degradation was identified testing single gene deletion mutants of MCMV, more precisely MCMV mutants, wherein m167, m168, m169 or m170 were deleted. MCMV mutants, wherein m167 has been deleted is referred to herein as MCMV-Am167 or Δm167, wherein m168 has been deleted is referred to herein as MCMV-Δm168 or Δm168, wherein m169 has been deleted is referred to herein as MCMV-Δm169 or Δm169 and wherein m170 has been deleted is referred to herein as MCMV-Δm170 or Δm170. Said MCMV mutants were kindly provided by Stipan Jonjic, Faculty of Medicine, University of Rijeka (Fig. 3A). The testing of said viral single gene mutants for miR-27a degradation lead to the initial finding that only the mutant lacking part of the m168 coding sequence not overlapping with m169 (389 nucleotides) did no longer result in miR-27a degradation (Fig. 3B). MiR-27a and miR-27b only differ in one nucleotide, said nucleotide is depicted being capitalized in the respective sequences of miR-27a and miR-27b in table 1.

Bioinformatic target predictions are well known to result in a large number of false positive miRNA binding sites. Therefore, it will be acknowledged by a person skilled in the art that target predictions for both DNA strands of the MCMV genome would result in hundreds of putative miRNA binding sites for miR-27a, many of which were located within essential genes which thus would have been very difficult to test. As the initial finding employing single gene deletion mutants, as has been outlined above, allowed to narrow down the putative region comprising a miR-27a miRNA binding site from >230000 nucleotides to 389 nucleotides, a miR-27a miRNA binding site could be identified in a putative MCMV transcript that binds miR-27a by applying a motif search using RNAhybrid (http://bibiserv.techfak.uni-bielefeld.de/rnahybrid/) and avoid this major problem. Both strands of the 389 nucleotides within the m168 coding region were parsed for miR-27a miRNA binding sites. This only resulted in the identification of a single putative miR-27a miRNA binding site at position 228159-228180 of the MCMV wild type genome according to Rawlinson et al. (Rawlinson et al., supra) on the minus strand, which corresponds to the 3'UTR of a predicted coding transcript m169. The interaction between miR-27a and m169 is indicated in Fig. 4A. The identity of the transcript including transcription start site, coding sequence including a single intron (which are only rarely found in viral genes), 3'-UTR and transcript end were determined by performing next-generation sequencing (SOLiD sequencing, Applied Biosystems) on total RNA prepared from MCMV infected fibroblasts at 24 and 48 hours post infection (Fig. 4B). Thereby, a small intron of 81 nucleotides located close to the 3'-end of the m169 coding sequence was identified. The putative binding for miR-27a is located within the 3'-UTR of this transcript. Finally, the present inventors validated that this binding site is indeed responsible for mediating the degradation of miR-27a by site directed mutagenesis (see Fig. 5 and Example 3)

### Example 3: Mutation of the binding site for miR-27a

The present inventors have found that the degradation of cellular miR-27a during MCMV infection is mediated by a single binding site in m 169. Mutation of said binding site as shown in Fig. 5A leads to a loss of regulation of degradation of miR-27a. Murine fibroblasts have been infected with MCMV wild type and MCMV-m169-mut at an MOI of 10 or left untreated as described in Example 1. After 48 hours of infection the levels of miR-27a were quantified using PCR and normalized to the level of a second cellular miRNA, more precisely miR-24. The infection of murine fibroblasts with MCMV wild type leads to a more than 5 fold loss after 4 h (Buck AH et al., Post-transcriptional regulation of miR-27 in murine cytomegalovirus infection, RNA. 2010 Feb;16(2):307-15. Epub 2010 Jan 4.) and an approximately 30 to 60-fold loss of miR-27a within 48 hours post infection as can be taken from Fig.1B/2B and 3B. Mutation of 3 bases within the binding site as depicted in Fig.5A, namely as in MCMV-m169-mut, abolished this effect. The present inventors also have found that reversion of said point mutations also restores said effect (Fig. 5B).

### Example 4: Retargeting of m169 to other cellular and viral miRNAs by target site replacement

MCMV wild type, MCMV-m169-miR16, m169-miR-M23-2, also referred to herein as MCMV-m169-miR-M23-2, and MCMV-m169-mut were generated as described in Example 1, thereby generating MCMV mutants targeting miR-16, namely m169-miR-16, or mcmv-miR-M23-2, namely m169-miR-M23-2, instead of miR-27a. Murine fibroblasts have been infected with MCMV wild type, MCMV-m169-miR16, MCMV-m169-miR-m23-2 and MCMV-m169-mut at an MOI of 10 using cells of 80% confluence. 48 hours post infection the levels of miRNAs miR-27a, miR-16 and mcmv-miR-M23-2 were quantified via PCR and normalized for miR-24 levels. For mcmv-miR-M23-2 miRNA levels were additionally normalized to MCMV wild-type infected cells to determine the effect of the retargeting on the levels of this viral miRNA. Fig. 6B shows the effect of the indicated viruses on the respective miRNAs they were designed to target. Retargeting of m169 to cellular miR-16 resulted in degradation of this very abundant cellular miRNA to a similar extent (>30-fold) as generally observed for miR-27a and wild-type MCMV. It is thus important to note that a regulation of levels of an miRNA to such high extend is absolutely unprecedented. A person skilled in the art will also acknowledge that the viral microRNA mcmv-miR-M23-2 is massively formed during the course of infection and that it is thus surprising that the levels of said microRNA are still approximately 8-fold reduced after 48 hours post infection compared to infection with MCMV wild type. As expected both m169-miR-16 and m169-miR-M23-2 were no longer able to mediate any degradation of miR-27a.

The features of the present invention disclosed in the specification, the claims, the sequence listing and/or the drawings may both separately and in any combination thereof be material for realizing the invention in various forms thereof.

## Claims

1. A nucleic acid molecule consisting of
(a) an miRNA binding site,
wherein the miRNA binding site is capable of binding a targeted miRNA and
wherein the miRNA binding site comprises about 20 to 25 nucleotides;
(b) a 3' flanking nucleotide sequence, wherein the 3' flanking nucleotide sequence is attached to the 3' end of the miRNA binding site and comprises the nucleotide sequence of SEQ ID NO: 1 and
(c) a 5' flanking nucleotide sequence, wherein the 5' flanking nucleotide sequence is attached to the 5' end of the miRNA binding site and comprises the nucleotide sequence of SEQ ID NO: 2.

2. The nucleic acid molecule according to claim 1, further comprising
(d) a coding nucleotide sequence, wherein the coding nucleotide sequence is attached to the 5' end of the 5' flanking nucleotide sequence and wherein the coding sequence codes for an amino acid sequence.

3. The nucleic acid molecule according to any one of claims 1 to 2, further comprising
(e) a non-translated transfer nucleotide sequence, which is contained within the coding sequence wherein the coding sequence is attached to the 5' end of the 5' flanking nucleotide sequence and wherein the non-translated transfer nucleotide sequence enhances the transfer of a nucleic acid molecule from a nucleus of a cell into the cytoplasma of the cell or influences the subcellular localization of a nucleic acid molecule within the cell, preferably the nucleic acid molecule is a nucleic acid molecule of any one of claims 1 to 2.

4. The nucleic acid molecule according to any one of claims 1 to 3 further comprising
(f) a non-coding nucleotide sequence, wherein the non-coding nucleotide sequence is attached to the 5' end of the coding sequence or the 5' end of the 5' flanking nucleotide sequence.

5. The nucleic acid molecule according to any one of claims 1 to 4, further comprising
(g) at least one promotor, wherein the at least one promoter is attached to the 5' of the non-coding nucleotide sequence, the 5' end of the coding sequence, or the 5' end of the 5' flanking group, and wherein the at least one promoter is operable linked to the 5' of the non-coding nucleotide sequence, the 5' end of the coding nucleotide sequence, or the 5' end of the 5' flanking nucleotide sequence so as to allow the transcription of one or several of the nucleotide sequences attached to the 3' end of the promoter.

6. The nucleic acid molecule according to any one of claims 1 to 5, wherein the miRNA binding site comprises
at positions 1 to 7 a nucleotide sequence A which is complementary to the targeted miRNA,
at positions 8 to 16 a nucleotide sequence B within which one, two, three, four, five, six, seven, eight or nine nucleotides are not complementary to the targeted miRNA; and
at positions 17 to the last position of the miRNA binding site a nucleotide sequence which is complementary to the targeted miRNA.

7. The nucleic acid molecule according to any one of claims 1 to 6, wherein the miRNA binding site comprises in 5'-3' direction a first stretch of consecutive nucleotides, a second stretch of consecutive nucleotides and a third stretch of consecutive nucleotides,
wherein the targeted micro RNA comprises in 3'-5' direction a stretch I of consecutive nucleotides, a stretch II of consecutive nucleotides and a stretch III of consecutive nucleotides,
wherein the first stretch of consecutive nucleotides of the miRNA biding site is essentially complementary to stretch I of the targeted miRNA,
wherein the second stretch of consecutive nucleotides of the miRNA biding site is not complementary to stretch II of the targeted miRNA,
wherein the third stretch of consecutive nucleotides of the miRNA biding site is essentially complementary to stretch III of the targeted miRNA.

8. The nucleic acid molecule according to any one of claims 1 to 7, wherein the nucleic acid molecule consists of an miRNA binding site as defined in any of the preceding claims, a nucleotide sequence according to SEQ ID NO: 9 attached to the 5' end of the miRNA binding site and a nucleotide sequence according to SEQ ID NO: 10 attached to the 3' end of the miRNA binding site.

9. The nucleic acid molecule according to any one of claims 1 to 8, wherein the nucleic acid molecule is for use in a method of inhibiting the function of an miRNA, preferably the inhibiting of the function is by degradation of the targeted the miRNA.

10. A nucleic acid library comprising a plurality of nucleic acid molecules, wherein each nucleic acid molecule of said nucleic acid library is a nucleic acid molecule according to any one of claims 1 to 9, wherein the nucleic acid molecules of the nucleic acid library differ in the sequence of the miRNA binding site.

11. A vector comprising at least one nucleic acid molecule according to any one of claims 1 to 9, wherein the vector is different from a vector comprising a murine cytomegalovirus genome.

12. A vector library comprising a plurality of vectors, wherein each vector of said vector library is a vector according to claim 11, wherein the vectors of the vector library differ in the sequence of the miRNA binding site.

13. A cell comprising a nucleic acid molecule according to any one of claims 1 to 9 and/or a vector according to claim 11.

14. A method for modulating the activity of an miRNA in a cell comprising the step of introducing into the cell a nucleic acid molecule according to any one of claims 1 to 9 and/or a vector according to claim 11, wherein, preferably, the targeted miRNA of the nucleic acid molecule is the miRNA the activity of which is to be modulated by said method.

15. A pharmaceutical composition comprising a nucleic acid molecule and/or a vector according to any one of claims 1 to 9 and /or a vector according to claim 11, and a pharmaceutically acceptable carrier.


SEQUENCE LISTING

<110>  Ludwig-Maximilians-Universität München

<120>  MICRORNA INHIBITING NUCLEIC ACID MOLECULE

<130>  U 10052 EP

<160>  44

<170>  PatentIn version 3.3

<210>  1
<211>  50
<212>  RNA
<213>  Artificial

<220>
<223>  synthetic

<400>  1
agaagucccu ucggaugucu gccugagaac ggucuccuca ucgggagaug                50

<210>  2
<211>  50
<212>  RNA
<213>  Artificial

<220>
<223>  synthetic

<400>  2
auccucgucc gccgucuccc cccguccgug aucuccccca gacgacgauc                50

<210>  3
<211>  693
<212>  RNA
<213>  Artificial

<220>
<223>  synthetic

<400>  3
agaagucccu ucggaugucu gccugagaac ggucuccuca ucgggagaug cuacuugaau      60

gcaauacgcu uuuuguuuuu uacgagaacg uguucuuuca cgcacauaca uccuucccuc     120

guguguccgu ccucgcaugc ucccuaguuc guagaugacc cguccuuagg uuucguuucu     180

guguaaacgg cucgugaagu uucgucuucu cggucgcccc gcuauagcuc cguccgagac     240

ccucaucgug guucccuaua uguucuaaga ccuccggguc gcggugcuaa caugccacua     300

aauccucacc gucaaccguc ggcugguccg aucggucgac ggccguccgu cuuccguguu     360

auccacuuag cauuaagauc ccugaccugu cccgaccuuu uuccucguaa ccuugaccgu     420

uccaugaccc cagcucccga augaccuuuu guccuucccc gauguuuuuc cccucgcccu     480

ucgucacauc gaacggucag uucguucccg ggguuguuuu ucuguuccga gcggacgucg 540

gggggucggu cucauccggg acgaccucuc aucuccuucc cgcuugagcu uucgccaguu 600

cgaaucaaua aaagcuauuc aaugauuuga caucucgucu gguacaucgu guuuuucugg 660

gugcuuaccc gcgucacaug gagggguggg uuc 693

<210> 4
<211> 104
<212> RNA
<213> Artificial

<220>
<223> synthetic

<400> 4
aacauccgac cgacugcccg ucuccuucgg aguagaaacg cuauccucgg agcgauccuc 60

guccgccguc uccccccguc cgugaucucc cccagacgac gauc 104

<210> 5
<211> 81
<212> RNA
<213> Artificial

<220>
<223> synthetic

<400> 5
guaggugccc cccgugauga gggaggcccg acgcuagauc uggcucggau gauaauuauu 60

aacguuguuu uugucugaca g 81

<210> 6
<211> 500
<212> RNA
<213> Artificial

<220>
<223> synthetic

<400> 6
gcucgcuggu cgagcgaccg cccggguuuc gagaugcccg ucguaacgcg acgccgguga 60

cgcaacgcga ccagcgugcc gcgucgcggg uucgcgcucg accuucgagc guuccuccgc 120

uguucgagug uugccacgua ccgcgcuccg gagaacgucg uucacggagg uguuacgcgg 180

agcgcaugac ugccgggcga gggcgccccg ccggcaugcu acaggcguua cggguugacc 240

gccgcgcagg uaccagguaa uaacgauguu cguguguucu gucuuuccag ccgaguucgu 300

gaucggucaa cuuucccugu gagcgcugcg ggaggccgag ugcgcgaucu cgcuccgcgc 360

gcucccucgc cguucguguc gcggaucgcu guccguuccc gcucaucggu cugacuucuc 420

ggaaaucccc gcucuuuugu cuccuccccc gcgcgggugc gcuugaagug uauaaaagcu 480

gaguguggag cggucgcagu 500

<210> 7
<211> 300
<212> RNA
<213> Artificial

<220>
<223> synthetic

<400> 7
gggcgccccg ccggcaugcu acaggcguua cggguugacc gccgcgcagg uaccagguaa 60

uaacgauguu cguguguucu gucuuuccag ccgaguucgu gaucggucaa cuuucccugu 120

gagcgcugcg ggaggccgag ugcgcgaucu cgcuccgcgc gcucccucgc cguucguguc 180

gcggaucgcu guccguuccc gcucaucggu cugacuucuc ggaaaucccc gcucuuuugu 240

cuccuccccc gcgcgggugc gcuugaagug uauaaaagcu gaguguggag cggucgcagu 300

<210> 8
<211> 2234
<212> RNA
<213> Artificial

<220>
<223> synthetic

<400> 8
gcucgcuggu cgagcgaccg cccggguuuc gagaugcccg ucguaacgcg acgccgguga 60

cgcaacgcga ccagcgugcc gcgucgcggg uucgcgcucg accuucgagc guuccuccgc 120

uguucgagug uugccacgua ccgcgcuccg gagaacgucg uucacggagg uguuacgcgg 180

agcgcaugac ugccgggcga gggcgccccg ccggcaugcu acaggcguua cggguugacc 240

gccgcgcagg uaccagguaa uaacgauguu cguguguucu gucuuuccag ccgaguucgu 300

gaucggucaa cuuucccugu gagcgcugcg ggaggccgag ugcgcgaucu cgcuccgcgc 360

gcucccucgc cguucguguc gcggaucgcu guccguuccc gcucaucggu cugacuucuc 420

ggaaaucccc gcucuuuugu cuccuccccc gcgcgggugc gcuugaagug uauaaaagcu 480

gaguguggag cggucgcagu cgacagcuuc acaucccggu gaccugucgc uuucgguggu 540

cucucucugc cugucugugg ugagcugugc uccggcgguu cccgcguccg ucaugagguc 600

caggucgugu gacacgcuaa auauggccau cgauaacggu gcauguauca cacaaacccu 660

ccguuccagu gaacacugcc ugcauaccuc ugucgcuaac gccgaagaag auuucugcga 720

aaaucagcug cugcgucguc uggcccacac cgugugcagu ggaaugacca ugcguaagac 780

cgaaaccaag uccugcuggu cugucagcuu cgcgcgcgac gaaguugcga uggauacgug 840

uaucaacacc aucuaaucag cuuuucgcuc cccgcgacgc cggacucgcg augagcaacg 900

cggucccguu cguccccuac gcggguaucg gccacaaccu ucgucgcugg gagcugcaau 960

gucguccgac cgacaccgag cuugcuacuu ucuccgccac uucaucguuc augucaccga 1020

aaguguucgg ugacgauagc cguugccguc uuuucuguug cugucccuac gauccccgcg 1080

aacccuacgg gccucacccg cugggugucg cauucuaccu cccugaccug gauucacguc 1140

gcaucgccgu guacuacacg aacguccguc cgaucagcga gcguccucug uucgcccagc 1200

gcgguuggag acgucguuau cagacguucg ccaagaucuu ggauacgcag gagcggcggg 1260

ccaccgaaag agguaggugc cccccgugau gagggaggcc cgacgcuaga ucuggcucgg 1320

augauaauua uuaacguugu uuuugucuga caggugacaa ccucuccggc uggcucucgg 1380

uccucgacgu gaauccuccu uccccuccgu ccugaaacau ccgaccgacu gcccgucucc 1440

uucggaguag aaacgcuauc cucggagcga uccucguccg ccgucucccc ccguccguga 1500

ucucccccag acgacgaucu gcggaauaau aagcugugaa aagaaguccc uucggauguc 1560

ugccugagaa cggucuccuc aucgggagau gcuacuugaa ugcaauacgc uuuuuguuuu 1620

uuacgagaac guguucuuuc acgcacauac auccuucccu cguguguccg uccucgcaug 1680

cucccuaguu cguagaugac ccguccuuag guuucguuuc uguguaaacg gcucgugaag 1740

uuucgucuuc ucggucgccc cgcuauagcu ccguccgaga cccucaucgu gguucccuau 1800

auguucuaag accuccgggu cgcggugcua acaugccacu aaauccucac cgucaaccgu 1860

cggcuggucc gaucggucga cggccguccg ucuuccgugu uauccacuua gcauuaagau 1920

cccugaccug ucccgaccuu uuuccucgua accuugaccg uuccaugacc ccagcucccg 1980

aaugaccuuu uguccuuccc cgauguuuuu ccccucgccc uucgucacau cgaacgguca 2040

guucguuccc gggguuguuu uucuguuccg agcggacguc ggggggucgg ucucauccgg 2100

gacgaccucu caucuccuuc ccgcuugagc uuucgccagu ucgaaucaau aaaagcuauu 2160

caaugauuug acaucucguc ugguacaucg uguuuuucug ggugcuuacc cgcgucacau 2220

ggaggggugg guuc 2234

<210> 9
<211> 1519
<212> RNA
<213> Artificial

<220>
<223> synthetic

<400> 9
gcucgcuggu cgagcgaccg cccggguuuc gagaugcccg ucguaacgcg acgccgguga 60

cgcaacgcga ccagcgugcc gcgucgcggg uucgcgcucg accuucgagc guuccuccgc 120

uguucgagug uugccacgua ccgcgcuccg gagaacgucg uucacggagg uguuacgcgg 180

agcgcaugac ugccgggcga gggcgccccg ccggcaugcu acaggcguua cggguugacc 240

gccgcgcagg uaccagguaa uaacgauguu cguguguucu gucuuuccag ccgaguucgu 300

gaucggucaa cuuucccugu gagcgcugcg ggaggccgag ugcgcgaucu cgcuccgcgc 360

gcucccucgc cguucguguc gcggaucgcu guccguuccc gcucaucggu cugacuucuc 420

ggaaaucccc gcucuuuugu cuccuccccc gcgcgggugc gcuugaagug uauaaaagcu 480

gaguguggag cggucgcagu cgacagcuuc acaucccggu gaccugucgc uuucgguggu 540

cucucucugc cugucugugg ugagcugugc uccggcgguu cccgcguccg ucaugagguc 600

caggucgugu gacacgcuaa auauggccau cgauaacggu gcauguauca cacaaacccu 660

ccguuccagu gaacacugcc ugcauaccuc ugucgcuaac gccgaagaag auuucugcga 720

aaaucagcug cugcgucguc uggcccacac cgugugcagu ggaaugacca ugcguaagac 780

cgaaaccaag uccugcuggu cugucagcuu cgcgcgcgac gaaguugcga uggauacgug 840

uaucaacacc aucuaaucag cuuuucgcuc cccgcgacgc cggacucgcg augagcaacg 900

cggucccguu cguccccuac gcggguaucg gccacaaccu ucgucgcugg gagcugcaau 960

gucguccgac cgacaccgag cuugcuacuu ucuccgccac uucaucguuc augucaccga 1020

aaguguucgg ugacgauagc cguugccguc uuuucuguug cugucccuac gauccccgcg 1080

aacccuacgg gccucacccg cugggugucg cauucuaccu cccugaccug gauucacguc 1140

gcaucgccgu guacuacacg aacguccguc cgaucagcga gcguccucug uucgcccagc 1200

gcgguuggag acgucguuau cagacguucg ccaagaucuu ggauacgcag gagcggcggg 1260

ccaccgaaag agguaggugc cccccgugau gagggaggcc cgacgcuaga ucuggcucgg 1320

augauaauua uuaacguugu uuuugucuga caggugacaa ccucuccggc uggcucucgg 1380

uccucgacgu gaauccuccu uccccuccgu ccugaaacau ccgaccgacu gcccgucucc 1440

uucggaguag aaacgcuauc cucggagcga uccucguccg ccgucucccc ccguccguga 1500

ucucccccag acgacgauc 1519

<210> 10
<211> 693
<212> RNA
<213> Artificial

<220>

<223>  synthetic

<400>  10
agaagucccu ucggaugucu gccugagaac ggucuccuca ucgggagaug cuacuugaau      60

gcaauacgcu uuuuguuuuu uacgagaacg uguucuuuca cgcacauaca uccuucccuc     120

guguguccgu ccucgcaugc ucccuaguuc guagaugacc cguccuuagg uuucguuucu     180

guguaaacgg cucgugaagu uucgucuucu cggucgcccc gcuauagcuc cguccgagac     240

ccucaucgug guucccuaua uguucuaaga ccuccggguc gcggugcuaa caugccacua     300

aauccucacc gucaaccguc ggcugguccg aucggucgac ggccguccgu cuuccguguu     360

auccacuuag cauuaagauc ccugaccugu cccgaccuuu uuccucguaa ccuugaccgu     420

uccaugaccc cagcucccga augaccuuuu guccuucccc gauguuuuuc cccucgcccu     480

ucgucacauc gaacggucag uucguucccg ggguuguuuu ucuguuccga gcggacgucg     540

gggggucggu cucauccggg acgaccucuc aucuccuucc cgcuugagcu uucgccaguu     600

cgaaucaaua aaagcuauuc aaugauuuga caucucgucu gguacaucgu guuuuucugg     660

gugcuuaccc gcgucacaug gagggguggg uuc                                  693

<210>  11
<211>  525
<212>  RNA
<213>  Artificial

<220>
<223>  synthetic

<400>  11
augagcaacg cggucccguu cguccccuac gcggguaucg gccacaaccu ucgucgcugg      60

gagcugcaau gucguccgac cgacaccgag cuugcuacuu ucuccgccac uucaucguuc     120

augucaccga aaguguucgg ugacgauagc cguugccguc uuuucuguug cugucccuac     180

gauccccgcg aacccuacgg gccucacccg cugggugucg cauucuaccu cccugaccug     240

gauucacguc gcaucgccgu guacuacacg aacguccguc cgaucagcga gcguccucug     300

uucgcccagc gcgguuggag acgucguuau cagacguucg ccaagaucuu ggauacgcag     360

gagcggcggg ccaccgaaag agguaggugc cccccgugau gagggaggcc cgacgcuaga     420

ucuggcucgg augauaauua uuaacguugu uuuugucuga caggugacaa ccucuccggc     480

uggcucucgg uccucgacgu gaauccuccu uccccuccgu ccuga                     525

<210>  12
<211>  720
<212>  RNA
<213>  Artificial

<220>
<223>  synthetic

<400>  12
auggugagca agggcgagga gcuguucacc gggguggugc ccauccuggu cgagcuggac      60

ggcgacguaa acggccacaa guucagcgug uccggcgagg gcgagggcga ugccaccuac     120

ggcaagcuga cccugaaguu caucugcacc accggcaagc ugcccgugcc cuggcccacc     180

cucgugacca cccugaccua cggcgugcag ugcuucagcc gcuaccccga ccacaugaag     240

cagcacgacu ucuucaaguc cgccaugccc gaaggcuacg uccaggagcg caccaucuuc     300

uucaaggacg acggcaacua caagacccgc gccgagguga aguucgaggg cgacacccug     360

gugaaccgca ucgagcugaa gggcaucgac uucaaggagg acggcaacau ccuggggcac     420

aagcuggagu acaacuacaa cagccacaac gucuauauca uggccgacaa gcagaagaac     480

ggcaucaagg ugaacuucaa gauccgccac aacaucgagg acggcagcgu gcagcucgcc     540

gaccacuacc agcagaacac ccccaucggc gacggccccg ugcugcugcc cgacaaccac     600

uaccugagca cccaguccgc ccugagcaaa gaccccaacg agaagcgcga ucacaugguc     660

cugcuggagu ucgugaccgc cgccgggauc acucucggca uggacgagcu guacaaguaa     720


<210>  13
<211>  444
<212>  RNA
<213>  Artificial

<220>
<223>  synthetic

<400>  13
augagcaacg cggucccguu cguccccuac gcggguaucg gccacaaccu ucgucgcugg      60

gagcugcaau gucguccgac cgacaccgag cuugcuacuu ucuccgccac uucaucguuc     120

augucaccga aaguguucgg ugacgauagc cguugccguc uuuucuguug cugucccuac     180

gauccccgcg aacccuacgg gccucacccg cugggugucg cauucuaccu cccugaccug     240

gauucacguc gcaucgccgu guacuacacg aacguccguc cgaucagcga gcguccucug     300

uucgcccagc gcgguuggag acgucguuau cagacguucg ccaagaucuu ggauacgcag     360

gagcggcggg ccaccgaaag aggugacaac cucuccggcu ggcucucggu ccucgacgug     420

aauccuccuu ccccuccguc cuga                                            444


<210>  14
<211>  1653
<212>  RNA
<213>  Artificial

<220>
<223>  synthetic

<400>  14
auggccgaug cuaagaacau uaagaagggc ccugcucccu ucuacccucu ggaggauggc      60

accgcuggcg agcagcugca caaggccaug aagagguaug cccuggugcc uggcaccauu     120

gccuucaccg augcccacau ugagguggac aucaccuaug ccgaguacuu cgagaugucu     180

gugcgccugg ccgaggccau gaagagguac ggccugaaca ccaaccaccg caucguggug     240

ugcucugaga acucucugca guucuucaug ccagugcugg gcgcccuguu caucggagug     300

gccguggccc cugcuaacga cauuuacaac gagcgcgagc ugcugaacag caugggcauu     360

ucucagccua ccgugguguu cgugucuaag aagggccugc agaagauccu gaacgugcag     420

aagaagcugc cuaucaucca gaagaucauc aucauggacu cuaagaccga cuaccagggc     480

uuccagagca uguacacauu cgugacaucu caucugccuc cuggcuucaa cgaguacgac     540

uucgugccag agucuuucga cagggacaaa accauugccc ugaucaugaa cagcucuggg     600

ucuaccggcc ugccuaaggg cguggcccug ccucaucgca ccgccugugu gcgcuucucu     660

cacgcccgcg acccuauuuu cggcaaccag aucauccccg acaccgcuau ucugagcgug     720

gugccauucc accacggcuu cggcauguuc accacccugg gcuaccugau uugcggcuuu     780

cggguggugc ugauguaccg cuucgaggag gagcuguucc ugcgcagccu gcaagacuac     840

aaaauucagu cugcccugcu ggugccaacc cuguucagcu ucuucgcuaa gagcacccug     900

aucgacaagu acgaccuguc uaaccugcac gagauugccu cuggcggcgc cccacugucu     960

aaggaggugg gcgaagccgu ggccaagcgc uuucaucugc caggcauccg ccagggcuac    1020

ggccugaccg agacaaccag cgccauucug auuaccccag agggcgacga caagccuggc    1080

gccgugggca agguggugcc auucuucgag gccaaggugg uggaccugga caccggcaag    1140

acccugggag ugaaccagcg cggcgagcug ugugugcgcg gcccuaugau uauguccggc    1200

uacgugaaua acccugaggc cacaaacgcc cugaucgaca aggacggcug gcugcacucu    1260

ggcgacauug ccuacuggga cgaggacgag cacuucuuca ucguggaccg ccugaagucu    1320

cugaucaagu acaagggcua ccagguggcc ccagccgagc uggagucuau ccugcugcag    1380

cacccuaaca uuuucgacgc cggaguggcc ggccugcccg acgacgaugc cggcgagcug    1440

ccugccgccg ucgucgugcu ggaacacggc aagaccauga ccgagaagga gaucguggac    1500

uauguggcca gccaggugac aaccgccaag aagcugcgcg gcggaguggu guucguggac    1560

gaggugccca agggccugac cggcaagcug gacgcccgca agauccgcga gauccugauc    1620

aaggcuaaga aaggcggcaa gaucgccgug uaa                                 1653

<210>  15
<211>  1734
<212>  RNA
<213>  Artificial

<220>
<223>  synthetic

<400>  15
cgacagcuuc acaucccggu gaccugucgc uuucgguggu cucucucugc cugucugugg      60

ugagcugugc uccggcgguu cccgcguccg ucaugagguc caggucgugu gacacgcuaa     120

auauggccau cgauaacggu gcauguauca cacaaacccu ccguuccagu gaacacugcc     180

ugcauaccuc ugucgcuaac gccgaagaag auuucugcga aaaucagcug cugcgucguc     240

uggcccacac cgugugcagu ggaaugacca ugcguaagac cgaaaccaag uccugcuggu     300

cugucagcuu cgcgcgcgac gaaguugcga uggauacgug uaucaacacc aucuaaucag     360

cuuuucgcuc cccgcgacgc cggacucgcg augagcaacg cggucccguu cguccccuac     420

gcggguaucg gccacaaccu ucgucgcugg gagcugcaau gucguccgac cgacaccgag     480

cuugcuacuu ucuccgccac uucaucguuc augucaccga aaguguucgg ugacgauagc     540

cguugccguc uuuucuguug cugucccuac gauccccgcg aacccuacgg gccucacccg     600

cugggugucg cauucuaccu cccugaccug gauucacguc gcaucgccgu guacuacacg     660

aacguccguc cgaucagcga gcguccucug uucgcccagc gcgguuggag acgucguuau     720

cagacguucg ccaagaucuu ggauacgcag gagcggcggg ccaccgaaag agguaggugc     780

cccccgugau gagggaggcc cgacgcuaga ucuggcucgg augauaauua uuaacguugu     840

uuuugucuga caggugacaa ccucuccggc uggcucucgg uccucgacgu gaauccuccu     900

uccccuccgu ccugaaacau ccgaccgacu gcccgucucc uucggaguag aaacgcuauc     960

cucggagcga uccucguccg ccgucucccc ccguccguga ucucccccag acgacgaucu    1020

gcggaauaau aagcugugaa aagaaguccc uucggauguc ugccugagaa cggucuccuc    1080

aucgggagau gcuacuugaa ugcaauacgc uuuuuguuuu uuacgagaac guguucuuuc    1140

acgcacauac auccuucccu cguguguccg uccucgcaug cucccuaguu cguagaugac    1200

ccguccuuag guuucguuuc uguguaaacg gcucgugaag uuucgucuuc ucggucgccc    1260

cgcuauagcu ccguccgaga cccucaucgu gguucccuau auguucuaag accuccgggu    1320

cgcggugcua acaugccacu aaauccucac cgucaaccgu cggcuggucc gaucggucga    1380

cggccguccg ucuuccgugu uauccacuua gcauuaagau cccugaccug ucccgaccuu    1440

uuuccucgua accuugaccg uuccaugacc ccagcucccg aaugaccuuu uguccuuccc    1500

cgauguuuuu ccccucgccc uucgucacau cgaacgguca guucguuccc gggguuguuu 1560

uucuguuccg agcggacguc ggggggucgg ucucauccgg gacgaccucu caucuccuuc 1620

ccgcuugagc uuucgccagu ucgaaucaau aaaagcuauu caaugauuug acaucucguc 1680

ugguacaucg uguuuuucug ggugcuuacc cgcgucacau ggaggggugg guuc 1734

<210> 16
<211> 22
<212> RNA
<213> Artificial

<220>
<223> synthetic

<400> 16
uggaguguga caaugguguu ug 22

<210> 17
<211> 22
<212> RNA
<213> Artificial

<220>
<223> synthetic

<400> 17
uggcucaguu cagcaggaac ag 22

<210> 18
<211> 23
<212> RNA
<213> Artificial

<220>
<223> synthetic

<400> 18
agcagcauug uacagggcua uga 23

<210> 19
<211> 22
<212> RNA
<213> Artificial

<220>
<223> synthetic

<400> 19
acaguagucu gcacauuggu ua 22

<210> 20
<211> 22

<212>  RNA
<213>  Artificial

<220>
<223>  synthetic

<400>  20
acagcaggca cagacaggca gu                                                22


<210>  21
<211>  22
<212>  RNA
<213>  Artificial

<220>
<223>  synthetic

<400>  21
uguaaacauc cuacacucag cu                                                22


<210>  22
<211>  22
<212>  RNA
<213>  Artificial

<220>
<223>  synthetic

<400>  22
uguaaacauc cccgacugga ag                                                22


<210>  23
<211>  23
<212>  RNA
<213>  Artificial

<220>
<223>  synthetic

<400>  23
caaagugcug uucgugcagg uag                                               23


<210>  24
<211>  22
<212>  RNA
<213>  Artificial

<220>
<223>  synthetic

<400>  24
aagugacggu gagauccagg cu                                                22


<210>  25
<211>  21

<212>  RNA
<213>  Artificial

<220>
<223>  synthetic

<400>  25
uucacagugg cuaaguuccg c                                                21


<210>  26
<211>  21
<212>  RNA
<213>  Artificial

<220>
<223>  synthetic

<400>  26
uucacagugg cuaaguucug c                                                21


<210>  27
<211>  104
<212>  DNA
<213>  Artificial

<220>
<223>  synthetic

<400>  27
tgaggagacc gttctcaggc agacatccga agggacttct ttccatagtt tattattccg      60

cagatcgtcg tctgggggag ataggatgac gacgataagt aggg                      104


<210>  28
<211>  25
<212>  DNA
<213>  Artificial

<220>
<223>  synthetic

<400>  28
caaccaatta accaattctg attag                                            25


<210>  29
<211>  24
<212>  DNA
<213>  Artificial

<220>
<223>  synthetic

<400>  29
tgaggagacc gttctcaggc agac                                             24

<210>  30
<211>  127
<212>  DNA
<213>  Artificial

<220>
<223>  synthetic

<400>  30
cctcggagcg atcctcgtcc gccgtctccc cccgtccgtg atctccccca gacgacgatc      60

tgcggaataa taaactatgg aaagaagtcc cttcggatgt ctcaaccaat taaccaattc     120

tgattag                                                               127


<210>  31
<211>  105
<212>  DNA
<213>  Artificial

<220>
<223>  synthetic

<400>  31
tgaggagacc gttctcaggc agacatccga agggacttct ttagcagcaa accctattgg      60

cgagatcgtc gtctggggga gataggatga cgacgataag taggg                     105


<210>  32
<211>  24
<212>  DNA
<213>  Artificial

<220>
<223>  synthetic

<400>  32
tgaggagacc gttctcaggc agac                                             24


<210>  33
<211>  128
<212>  DNA
<213>  Artificial

<220>
<223>  synthetic

<400>  33
cctcggagcg atcctcgtcc gccgtctccc cccgtccgtg atctccccca gacgacgatc      60

tcgccaatag ggtttgctgc taaagaagtc ccttcggatg tctcaaccaa ttaaccaatt     120

ctgattag                                                              128


<210>  34
<211>  104

<212>  DNA
<213>  Artificial

<220>
<223>  synthetic

<400>  34
tgaggagacc gttctcaggc agacatccga agggacttct tatgggggcg tttacaagcg     60

gagatcgtcg tctgggggag ataggatgac gacgataagt aggg                     104


<210>  35
<211>  24
<212>  DNA
<213>  Artificial

<220>
<223>  synthetic

<400>  35
tgaggagacc gttctcaggc agac                                            24


<210>  36
<211>  127
<212>  DNA
<213>  Artificial

<220>
<223>  synthetic

<400>  36
cctcggagcg atcctcgtcc gccgtctccc cccgtccgtg atctccccca gacgacgatc     60

tccgcttgta aacgccccca taagaagtcc cttcggatgt ctcaaccaat taaccaattc    120

tgattag                                                              127


<210>  37
<211>  104
<212>  DNA
<213>  Artificial

<220>
<223>  synthetic

<400>  37
tgaggagacc gttctcaggc agacatccga agggacttct tttcacagct tattattccg     60

cagatcgtcg tctgggggag ataggatgac gacgataagt aggg                     104


<210>  38
<211>  24
<212>  DNA
<213>  Artificial

<220>

<223>  synthetic

<400>  38
tgaggagacc gttctcaggc agac                                              24


<210>  39
<211>  128
<212>  DNA
<213>  Artificial

<220>
<223>  synthetic

<400>  39
cctcggagcg atcctcgtcc gccgtctccc cccgtccgtg atctccccca gacgacgatc      60

tgcggaataa taagctgtga aaaagaagtc ccttcggatg tctcaaccaa ttaaccaatt     120

ctgattag                                                               128


<210>  40
<211>  22
<212>  DNA
<213>  Artificial

<220>
<223>  synthetic

<400>  40
tggctcagtt cagcaggaac ag                                                22


<210>  41
<211>  21
<212>  DNA
<213>  Artificial

<220>
<223>  synthetic

<400>  41
ttcacagtgg ctaagttccg c                                                 21


<210>  42
<211>  22
<212>  DNA
<213>  Artificial

<220>
<223>  synthetic

<400>  42
tagcagcacg taaatattgg cg                                                22


<210>  43
<211>  23

<212>  DNA
<213>  Artificial

<220>
<223>  synthetic

<400>  43
atgggggcct cggtcaagcg gaa                                              23


<210>  44
<211>  22
<212>  RNA
<213>  Artificial

<220>
<223>  synthetic

<400>  44
ugcggaauaa uaagcuguga aa                                               22